(19) Europäisches Patentamt
European Patent Office
Office européen des brevets

(11) EP 1 589 028 A2

(12) # EUROPÄISCHE PATENTANMELDUNG

(43) Veröffentlichungstag:
**26.10.2005 Patentblatt 2005/43**

(51) Int Cl.[7]: **C07H 21/00**, C07H 19/056, C12N 15/11, C12Q 1/68

(21) Anmeldenummer: **05009923.3**

(22) Anmeldetag: **26.04.1995**

(84) Benannte Vertragsstaaten:
**AT BE CH DE DK ES FR GB GR IE IT LI LU NL PT SE**

(30) Priorität: **02.05.1994 DE 4415370**

(62) Dokumentnummer(n) der früheren Anmeldung(en) nach Art. 76 EPÜ:
**95106230.6 / 0 680 969**

(71) Anmelder: **Aventis Pharma Deutschland GmbH**
**65926 Frankfurt am Main (DE)**

(72) Erfinder:
- **Seela, Frank**
  **49076 Osnabrück (DE)**
- **Lampe, Sigrid**
  **49626 Berge/Hekese (DE)**

Bemerkungen:
Diese Anmeldung ist am 06 - 05 - 2005 als Teilanmeldung zu der unter INID-Kode 62 erwähnten Anmeldung eingereicht worden.

(54) **Modifizierte Oligonukleotide, deren Herstellung sowie deren Verwendung**

(57) Die Erfindung betrifft neue modifizierte Oligonukleotide, die mindestens eine 8-Azapurinbase aufweisen und stabilere Hybdridisierungskomplexe mit Nukleinsäuren bilden;
Verfahren zu deren Herstellung sowie deren Verwendung als Inhibitoren der Genexpression, als Sonden zum Nachweis von Nukleinsäuren, als Hilfsmittel in der Molekularbiologie und als Arzneimittel oder Diagnostikum.

EP 1 589 028 A2

SEQUENZPROTOKOLL

(1) ALLGEMEINE INFORMATION:

(i) ANMELDER:
(A) NAME: Hoechst Aktiengesellschaft
(B) STRASSE: -
(C) ORT: Frankfurt am Main
(D) BUNDESLAND: -
(E) LAND: Deutschland
(F) POSTLEITZAHL: 65926
(G) TELEPHON: 069-305-6031
(H) TELEFAX: 069-35 7175
(I) TELEX: 41234700 hod

(ii) ANMELDETITEL: Modifizierte Oligonucleotide, deren Herstellung sowie deren Verwendung

(iii) ANZAHL DER SEQUENZEN: 30

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: HIV

(ix) MERKMALE:
(A) NAME/SCHLÜSSEL: exon
(B) LAGE: 1..20

(xi) SEQUENZBESCHREIBUNG: SEQ ID NO: 1:

ACACCCAATT CTGAAAATGG 20

(2) INFORMATION ZU SEQ ID NO: 2:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: HIV

(ix) MERKMALE:
(A) NAME/SCHLÜSSEL: exon
(B) LAGE: 1..20

(xi) SEQUENZBESCHREIBUNG: SEQ ID NO: 2:

AGGTCCCTGT TCGGGCGCCA 20

(2) INFORMATION ZU SEQ ID NO: 3:

(i) SEQUENZ CHARAKTERISTIKA:
(A) LÄNGE: 28 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: HIV

(ix) MERKMALE:
(A) NAME/SCHLÜSSEL: exon
(B) LAGE: 1..28

(xi) SEQUENZBESCHREIBUNG: SEQ ID NO: 3:

GTCGACACCC AATTCTGAAA ATGGATAA 28

(2) INFORMATION ZU SEQ ID NO: 4:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: HIV

(ix) MERKMALE:
(A) NAME/SCHLÜSSEL: exon
(B) LAGE: 1..25

(xi) SEQUENZBESCHREIBUNG: SEQ ID NO: 4:

GCTATGTCGA CACCCAATTC TGAAA 25

(2) INFORMATION ZU SEQ ID NO: 5:

(i) SEQUENZ CHARAKTERISTIKA:
(A) LÄNGE: 31 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: HIV

(ix) MERKMALE:
(A) NAME/SCHLÜSSEL: exon
(B) LAGE: 1..31

(xi) SEQUENZBESCHREIBUNG: SEQ ID NO: 5:

TCGTCGCTGT CTCCGCTTCT TCTTCCTGCC A 31

(2) INFORMATION ZU SEQ ID NO: 6:

(i) SEQUENZ CHARAKTERISTIKA:
(A) LÄNGE: 31 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: HIV

(ix) MERKMALE:
(A) NAME/SCHLÜSSEL: exon
(B) LAGE: 1..31

(xi) SEQUENZBESCHREIBUNG: SEQ ID NO: 6:

CTGTCTCCGC TTCTTCTTCC TGCCATAGGA G 31

(2) INFORMATION ZU SEQ ID NO: 7:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: HSV-1

(ix) MERKMALE:
(A) NAME/SCHLÜSSEL: exon
(B) LAGE: 1..20

(xi) SEQUENZBESCHREIBUNG: SEQ ID NO: 7:

GCGGGGCTCC ATGGGGGTCG 20

(2) INFORMATION ZU SEQ ID NO: 8:

(i) SEQUENZ CHARAKTERISTIKA:
(A) LÄNGE: 15 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..15
(D) SONSTIGE ANGABEN: /note= "c-Ha-ras"

(xi) SEQUENZBESCHREIBUNG: SEQ ID NO: 8:

CAGCTGCAAC CCAGC 15

(2) INFORMATION ZU SEQ ID NO: 9:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..21
(D) SONSTIGE ANGABEN: /note= "c-myc"

(xi) SEQUENZBESCHREIBUNG: SEQ ID NO: 9:

GGCTGCTGGA GCGGGGCACA C 21

(2) INFORMATION ZU SEQ ID NO: 10:

(i) SEQUENZ CHARAKTERISTIKA:
(A) LÄNGE: 15 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..15
(D) SONSTIGE ANGABEN: /note= "c-myc"

(xi) SEQUENZBESCHREIBUNG: SEQ ID NO: 10:

AACGTTGAGG GGCAT 15

(2) INFORMATION ZU SEQ ID NO: 11:

(i) SEQUENZ CHARAKTERISTIKA:
(A) LÄNGE: 15 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..15
(D) SONSTIGE ANGABEN: /note= "c-myb"

(xi) SEQUENZBESCHREIBUNG: SEQ ID NO: 11:

CACGTTGAGG GGCAT 15

(2) INFORMATION ZU SEQ ID NO: 12:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..18
(D) SONSTIGE ANGABEN: /note= "c-myb"

(xi) SEQUENZBESCHREIBUNG: SEQ ID NO: 12:

GTGCCGGGGT CTTCGGGC 18

(2) INFORMATION ZU SEQ ID NO: 13:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Maus

(ix) MERKMALE:
(A) NAME/SCHLÜSSEL: exon
(B) LAGE: 1..18
(D) SONSTIGE ANGABEN: /note= "c-myb"

(xi) SEQUENZBESCHREIBUNG: SEQ ID NO: 13:

GTGTCGGGGT CTCCGGGC 18

(2) INFORMATION ZU SEQ ID NO: 14:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..21
(D) SONSTIGE ANGABEN: /note= "c-fos"

(xi) SEQUENZBESCHREIBUNG: SEQ ID NO: 14:

GGAGAACATC ATGGTCGAAA G 21

(2) INFORMATION ZU SEQ ID NO: 15:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..22
(D) SONSTIGE ANGABEN: /note= "c-fos"

(xi) SEQUENZBESCHREIBUNG: SEQ ID NO: 15:

CCCGAGAACA TCATGGTCGA AG 22

(2) INFORMATION ZU SEQ ID NO: 16:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..20
(D) SONSTIGE ANGABEN: /note= "c-fos"

(xi) SEQUENZBESCHREIBUNG: SEQ ID NO: 16:

GGGGAAAGCC CGGCAAGGGG 20

(2) INFORMATION ZU SEQ ID NO: 17:

(i) SEQUENZ CHARAKTERISTIKA:
(A) LÄNGE: 20 Basenpaare
(B) ART: Nukleinsäure

(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..20
(D) SONSTIGE ANGABEN: /note= "p-120"

(xi) SEQUENZBESCHREIBUNG: SEQ ID NO: 17:

CACCCGCCTT GGCCTCCCAC 20

(2) INFORMATION ZU SEQ ID NO: 18:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..18
(D) SONSTIGE ANGABEN: /note= "EGF-Rezeptor"

(xi) SEQUENZBESCHREIBUNG: SEQ ID NO: 18:

GGGACTCCGG CGCAGCGC 18

(2) INFORMATION ZU SEQ ID NO: 19:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..20
(D) SONSTIGE ANGABEN: /note= "EGF-Rezeptor"

(xi) SEQUENZBESCHREIBUNG: SEQ ID NO: 19:

GGCAAACTTT CTTTTCCTCC 20

(2) INFORMATION ZU SEQ ID NO: 20:

(i) SEQUENZ CHARAKTERISTIKA:
(A) LÄNGE: 19 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..19
(D) SONSTIGE ANGABEN: /note= "p53 Tumorsuppressor"

(xi) SEQUENZBESCHREIBUNG: SEQ ID NO: 20:

GGGAAGGAGG AGGATGAGG 19

(2) INFORMATION ZU SEQ ID NO: 21:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..21
(D) SONSTIGE ANGABEN: /note= "p53 Tumorsuppressor"

(xi) SEQUENZBESCHREIBUNG: SEQ ID NO: 21:

GGCAGTCATC CAGCTTCGGA G 21

(2) INFORMATION ZU SEQ ID NO: 22:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..18
(D) SONSTIGE ANGABEN: /note= "cdc2-Kinase"

(xi) SEQUENZBESCHREIBUNG: SEQ ID NO: 22:

GTCTTCCATA GTTACTCA 18

(2) INFORMATION ZU SEQ ID NO: 23:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..18
(D) SONSTIGE ANGABEN: /note= "PCNA (proliferating cell nuclear antigen)"

(xi) SEQUENZBESCHREIBUNG: SEQ ID NO: 23:

GATCAGGCGT GCCTCAAA 18

(2) INFORMATION ZU SEQ ID NO: 24:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..18
(D) SONSTIGE ANGABEN: /note= "VLA-4"

(xi) SEQUENZBESCHREIBUNG: SEQ ID NO: 24:

GCAGTAAGCA TCCATATC 18

(2) INFORMATION ZU SEQ ID NO: 25:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..20
(D) SONSTIGE ANGABEN: /note= "ICAM"

(xi) SEQUENZBESCHREIBUNG: SEQ ID NO: 25:

CCCCCACCAC TTCCCCTCTC 20

(2) INFORMATION ZU SEQ ID NO: 26:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..20
(D) SONSTIGE ANGABEN: /note= "ICAM"

(xi) SEQUENZBESCHREIBUNG: SEQ ID NO: 26:

CTCCCCCACC ACTTCCCCTC 20

(2) INFORMATION ZU SEQ ID NO: 27:

(i) SEQUENZ CHARAKTERISTIKA:
(A) LÄNGE: 19 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..19
(D) SONSTIGE ANGABEN: /note= "ICAM"

(xi) SEQUENZBESCHREIBUNG: SEQ ID NO: 27:

GCTGGGAGCC ATAGCGAGG 19

(2) INFORMATION ZU SEQ ID NO: 28:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..21
(D) SONSTIGE ANGABEN: /note= "ELAM-1"

(xi) SEQUENZBESCHREIBUNG: SEQ ID NO: 28:

ACTGCTGCCT CTTGTCTCAG G 21

(2) INFORMATION ZU SEQ ID NO: 29:

(i) SEQUENZ CHARAKTERISTIKA:
(A) LÄNGE: 22 Basenpaare
(B) ART: Nukleinsäure

(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: JA

(vi) URSPRÜNLICHE HERKUNFT:
(A) ORGANISMUS: Mensch

(ix) MERKMALE:
(A) NAME/SCHLÜSSEL: exon
(B) LAGE: 1..22
(D) SONSTIGE ANGABEN: /note= "ELAM-1"

(xi) SEQUENZBESCHREIBUNG: SEQ ID NO: 29:

CAATCAATGA CTTCAAGAGT TC 22

(2) INFORMATION ZU SEQ ID NO: 30:

(i) SEQUENZ CHARAKTERISTIKA:
(A) LÄNGE: 11 Basenpaare
(B) ART: Nukleinsäure
(C) STRANGFORM: Einzel
(D) TOPOLOGIE: linear

(ii) ART DES MOLEKÜLS: DNS

(iii) HYPOTHETISCH: JA

(iii) ANTISENSE: JA

(ix) MERKMALE:
(A) NAME/SCHLÜSSEL: -
(B) LAGE: 1..11
(D) SONSTIGE ANGABEN: /note= "N = 8-Azaguanin"

(xi) SEQUENZBESCHREIBUNG: SEQ ID NO: 30:

GTANAATTCT AG 12

## Beschreibung

Die vorliegende Erfindung betrifft neue, modifizierte Basen enthaltende Oligonucleotide mit wertvollen physikalischen, biologischen und pharmakologischen Eigenschaften , ein Verfahren zu deren Herstellung sowie deren Verwendung als Inhibitoren der Genexpression (Antisense Oligonukleotide, Ribozyme, Sense Oligonukleotide und Triplex Forming Oligonukleotide), als Sonden zum Nachweis von Nucleinsäuren, als Hilfsmittel in der Molekularbiologie und als Arzneimittel oder Diagnostikum.

Aus der Literatur sind zahlreiche chemische Modifikationen von Oligonukleotiden bekannt. Diese Modifikation können das Zucker-Phosphat-Gerüst oder die Nucleobasen betreffen. Eine Übersicht über den Stand der Technik geben beispielsweise Uhlmann & Peyman, Chem. Rev. 1990, 90, 543 und Milligan et al., J. Med. Chem. 1993, 36, 1923].

Eine chemische Modifikation von Oligonukleotiden ist in der Regel erforderlich, da unmodifizierte Oligonukleotide sehr rasch durch nukleolytische Aktivitäten sowohl in Zellen als auch im Zellkulturmedium abgebaut werden. Die Stabilisierung gegen nucleolytischen Abbau kann durch Ersatz des Zucker-Phosphat-Rückgrats oder durch Modifikation der Phosphatbrücke, des Zuckerbausteins oder der Nucleobase erfolgen [ Milligan et al., supra und Uhlmann & Peyman, supra].

Neben Modifikationen, die zu Oligonukleotiden mit einer erhöhten Stabilität gegenüber einem nukleolytischen Abbau führen, sind Modifikationen von Interesse, die das Hybridisierungsverhalten der modifizierten Oligonukleotide dahingehend verändern, daß diese z. B. stabilere Hybridisierungskomplexe (Duplexe) mit intrazellulären Nukleinsäuren (sogenannte Target-Nukleinsäuren) bilden können. Eine Veränderung der Hybridisierungseigenschaften von Oligonukleotiden ist z.B. durch Modifikation der Basen möglich. Die veränderten Hybridisierungseigenschaften solch modifizierter Oligonukleotide sind z.B. an der - im Vergleich zu den unmodifizierten Oligonukleotiden - veränderten Schmelztemperatur ($T_m$-Wert) der Duplexe zu erkennen.

So werden in der PCT-Anmeldung WO 92/002258 pyrimidin-modifizierte Oligonukleotide beschrieben, die allerdings nicht eine erhöhte sondern eine verminderte Bindungsaffinität zu einzel- und doppelsträngigen Targetnukleinsäuren aufweisen. Aus der PCT-Anmeldung WO 93/10820 sind jedoch auch Oligonukleotide bekannt, die modifizierte Uracil- bzw. Cytosinbasen enthalten und im Vergleich zu den nicht modifizierten Oligonukleotiden stabilere Duplex- oder Triplexstrukturen mit den Targetnukleinsäuren ausbilden können. Die Hybridisierungseigenschaften von synthetischen Dodecameroligonucleotiden, die das Basenanalogon Pyridopyrimidin enthalten, wurden ebenfalls untersucht [Inoue et al.(1985), Nucleic Acid Res., 13, 7119-7129]. Verbesserte Hybridisierungseigenschaften wurden auch für Oligonukleotide beschrieben, die das Basenanalogon 2-Aminoadenin enthalten [Chollet et al, (1988), Nucleic Acid Research, 16, 305-317].

Aufgabe der vorliegenden Erfindung ist es daher, neue Oligonukleotide mit vorteilhaften Eigenschaften zur Verfügung zu stellen.

Es wurde nun überraschend gefunden, daß Oligonukleotide, die mindestens eine 8-Aza-Purinbase, beispielsweise das 8-Azaguanin bzw. das 8-Azaadenin enthalten, deutlich stabilere Hybridisierungskomplexe mit den Targetnukleinsäuren bilden als vergleichbare Oligonukleotide, die die unmodifizierten Purinbasen aufweisen.

Die Erfindung betrifft somit Oligonukleotide der Formel I

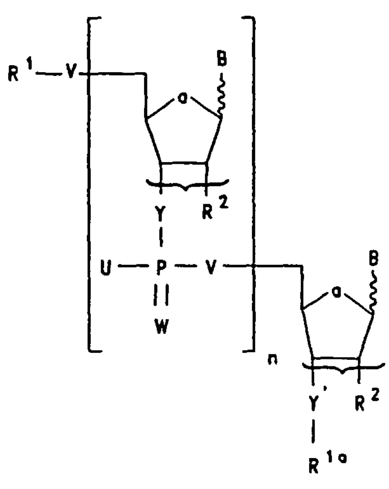

(I)

sowie deren physiologisch verträglichen Salze worin

$R^1$ Wasserstoff, $C_1$-$C_{18}$-Alkyl, $C_2$-$C_{18}$-Alkenyl, $C_2$-$C_{18}$-Alkinyl, $C_2$-$C_{18}$-Alkylcarbonyl, $C_3$-$C_{19}$-Alkenylcarbonyl, $C_3$-$C_{19}$-Alkinylcarbonyl, ($C_6$-$C_{14}$)-Aryl-($C_1$-$C_8$)-alkyl, eine in der Nukleotidchemie übliche Schutzgruppe oder einen Rest der Formel IIa

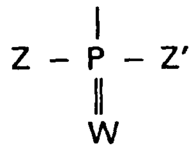 (IIa)

bedeutet;

$R^{1a}$ Wasserstoff, $C_1$-$C_{18}$-Alkyl, $C_2$-$C_{18}$-Alkenyl, $C_2$-$C_{18}$-Alkinyl, $C_2$-$C_{18}$-Alkylcarbonyl, $C_3$-$C_{19}$-Alkenylcarbonyl, $C_3$-$C_{19}$-Alkinylcarbonyl, ($C_6$-$C_{14}$)-Aryl-($C_1$-$C_8$)-alkyl, oder einen Rest der Formel IIb

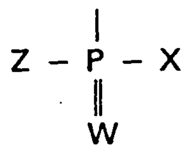 (IIb)

bedeutet;

$R^2$ Wasserstoff, Hydroxy, $C_1$-$C_{18}$-Alkoxy, $C_1$-$C_6$-Alkenyloxy, insbesondere Allyloxy, Halogen, Azido oder $NH_2$ bedeutet;

a für Oxy oder Methylen steht;

n eine ganze Zahl von 3 bis 99 bedeutet;

W Oxo, Thioxo oder Selenoxo bedeutet;

V Oxy, Sulfandiyl oder Imino bedeutet;

Y Oxy, Sulfandiyl, Imino oder Methylen bedeutet;

Y' Oxy, Sulfandiyl, Imino, $(CH_2)_m$ oder $V(CH_2)_m$ ist, worin

m eine ganze Zahl von 1 bis 18 bedeutet;

X Hydroxy oder Mercapto bedeutet;

U Hydroxy, Mercapto, SeH, $C_1$-$C_{18}$-Alkoxy, $C_1$-$C_{18}$-Alkyl, $C_6$-$C_{20}$-Aryl, ($C_6$-$C_{14}$)-Aryl-($C_1$-$C_8$)-alkyl, $NHR^3$, $NR^3R^4$ oder einen Rest der Formel III

$$(OCH_2CH_2)_pO(CH_2)_qCH_2R^5 \qquad \text{(III)}$$

bedeutet, worin

$R^3$ $C_1$-$C_{18}$-Alkyl, $C_6$-$C_{20}$-Aryl, ($C_6$-$C_{14}$)-Aryl-($C_1$-$C_8$)-alkyl, 2-$(CH_2)_c$-$[NH(CH_2)_c]_d$-$NR^6R^6$, worin c eine ganze Zahl von 2 bis 6 und d eine ganze Zahl von 0 bis 6 ist, und $R^6$ unabhängig voneinander Wasserstoff oder $C_1$-$C_6$-Alkyl oder $C_1$-$C_4$-Alkoxy-$C_1$-$C_6$-alkyl ist;

$R^4$ $C_1$-$C_{18}$-Alkyl, $C_6$-$C_{20}$-Aryl oder ($C_6$-$C_{10}$)-Aryl-($C_1$-$C_8$)-alkyl bedeutet oder im Falle von $NR^3R^4$ zusammen mit $R^3$ und dem sie tragenden Stickstoffatom einen 5-6-gliedrigen heterozyklischen Ring bedeutet, der zusätzlich ein weiteres Heteroatom aus der Reihe O, S, N enthalten kann,

p eine ganze Zahl von 1 bis 100 ist,

q eine ganze Zahl von 0 bis 22 ist,

$R^5$ Wasserstoff oder eine funktionelle Gruppe wie beispielsweise Hydroxy, Amino, $C_1$-$C_{18}$-Alkylamino, COOH, $CONH_2$, COO($C_1$-$C_4$)-Alkyl oder Halogen bedeutet;

Z und Z' unabhängig voneinander

Hydroxy, Mercapto, SeH, $C_1$-$C_{22}$-Alkoxy, -O-$(CH_2)_b$-$NR^6R^7$, worin b eine ganze Zahl von 1 bis 6 ist, und $R^7$ $C_1$-$C_6$-Alkyl ist oder $R^6$ und $R^7$ zusammen mit dem sie tragenden Stickstoffatom einen 3-6 gliedrigen Ring bilden, $C_1$-$C_{18}$-Alkyl, $C_6$-$C_{20}$-Aryl, ($C_6$-$C_{14}$)-Aryl-($C_1$-$C_8$)-alkyl, ($C_6$-$C_{14}$)-Aryl-($C_1$-$C_8$)-alkoxy, wobei Aryl auch Heteroaryl bedeutet und Aryl gegebenenfalls durch 1, 2 oder 3 gleiche oder verschiedene Reste aus der Reihe Carboxy, Amino, Nitro, $C_1$-$C_4$-Alkylamino, $C_1$-$C_6$-Alkoxy, Hydroxy, Halogen und Cyano sub-

stituiert ist, $C_1$-$C_{18}$-Alkylmercapto, $NHR^3$, $NR^3R^4$, einen Rest der Formel III oder eine Gruppe bedeuten, die die intrazelluläre Aufnahme begünstigt oder als Markierung einer DNA-Sonde dient oder bei der Hybridisierung des Oligonucleotidanalogons an die Target-Nucleinsäure diese unter Bindung, Quervernetzung oder Spaltung angreift, und

die geschweifte Klammer andeutet, daß sich $R^2$ und der benachbarte Phosphoryl-Rest in 2'- und 3'-Stellung oder auch umgekehrt in 3'- und 2'-Stellung befinden können,

wobei jedes Nucleotid in seiner D- bzw. L-Konfiguration vorliegen kann und sich die Base B in α- bzw. β-Stellung befinden kann, wobei unabhängig voneinander für eine in der Nukleotidchemie übliche Base, beispielsweise für natürliche Basen wie Adenin, Cytosin, Thymin, Guanin, Uracil, Hypoxanthin oder unnatürliche Basen wie beispielsweise Purin, 8-Azapurin, 2.6-Diaminopurin, 7-Deazaadenin, 7-Deazaguanin, $N^4N^4$-Ethancytosin, $N^6N^6$-Ethano-2.6-diaminopurin, Pseudoisocytosin, 5-Methylcytosin, 5-Fluoruracil, 5-($C_3$-$C_6$)-Alkinyluracil, 5-($C_3$-$C_6$)-Alkinylcytosin oder deren Prodrugformen steht, wobei mindestens ein B eine Base der Formel IV bedeutet

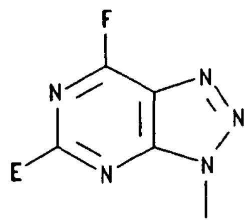

(IV)

worin E und F unabhängig voneinander H, OH oder $NH_2$ bedeuten.

Bevorzugt sind Verbindungen der Formel I, worin E gleich $NH_2$ und F gleich OH ist oder E gleich H und F gleich $NH_2$ ist.
Besonders bevorzugt sind Verbindungen der Formel I, worin E gleich $NH_2$ und F gleich OH ist.
Bevorzugt sind auch Verbindungen der Formel I, bei denen sich die Base am Zucker in β-Stellung befindet, die Nucleoside in der D-Konfiguration vorliegen, $R^2$ sich in 2'-Stellung befindet und a für Oxy steht.

Die erfindungsgemäßen Oligonukleotide weisen im Vergleich zu den natürlichen Oligonukleotiden bei der Anlagerung an komplementäre Nukleinsäuren (Targetnukleinsäuren) eine verbesserte Bindungsaffinität auf. Für die therapeutische Nutzung dieser Oligonukleotide ist es vorteilhaft, wenn in diese zusätzliche Modifikationen, beispielsweise des Phosphatrückgrats, der Ribose-Einheit oder der Oligonukleotid-Enden eingeführt werden [J.S. Cohen, Topics in Molecular and Structural Biology 12 (1989) Macmillan Press, E. Uhlmann et al., supra].
Durch ansich bekannte Modifikationen des Zucker-Phosphat-Rückrats werden die erfindungsgemäßen Oligonukleotide beispielsweise noch besser gegen den Angriff von Nukleasen geschützt, was vorteilhaft ist.

Bevorzugt sind daher auch Verbindungen der Formel I, worin V, Y, Y' und W die Bedeutung von Thioxo, Selenoxo, Oxy, Oxo, Sulfandiyl, Imino oder Methylen haben und U die Bedeutung von Hydroxy, Mercapto oder Methyl hat. Ganz besonders bevorzugt sind diese , falls $R^2$ zusätzlich Hydroxy oder Wasserstoff, insbesondere Wasserstoff bedeutet.

Eine bevorzugte Ausführungsform stellen auch Verbindungen der Formel I dar, in denen $R^1$ und $R^{1a}$ Wasserstoff bedeuten.

Ganz besonders bevorzugt sind Verbindungen der Formel I, in denen $R^1$ und/oder $R^{1a}$ Wasserstoff, $R^2$ Hydroxy oder Wasserstoff, U Hydroxy oder Mercapto und V, Y, Y' und W die Bedeutung von Thioxo, Oxy, Oxo oder Hydroxy haben.

Unter den in der Nukleotidchemie üblichen Schutzgruppen sind beispielsweise Amino-, Hydroxyl- oder andere Schutzgruppen zu verstehen, wie sie in [E.Sonveaux, 1986, Bioorganic Chemistry, 14, 274-325 oder S.L. Beaucage et al., 1992, Tetrahedron, 48, 2223-2311] beschrieben werden.

Alkyl, Alkenyl und Alkinyl können geradkettig oder verzweigt sein.

Unter Cycloalkyl werden auch Alkyl-substituierte Ringe verstanden.

($C_6$-$C_{20}$)-Aryl ist beispielsweise Phenyl, Naphthyl oder Biphenyl, vorzugsweise Phenyl.

Unter Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliederigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Unter physiologisch verträglichen Salzen von Verbindungen der Formel (I) versteht man sowohl anorganische als auch organische Salze, wie sie in Remington's Pharmaceutical Sciences (17. Auflage, Seite 1418 (1985)) beschrieben sind.

Aufgrund der physikalischen und chemischen Stabilität und der Löslichkeit sind für saure Gruppen unter anderem Natrium-, Kalium-, Calcium- und Ammoniumsalze bevorzugt.

Die Erfindung ist nicht auf α- und β-D- bzw. L-Ribofuranoside, α- und β-D- bzw. L- Desoxyribofuranoside und entsprechende carbocyclische Fünfringanaloga beschränkt, sondern gilt auch für Oligonucleotidanaloga, die aus anderen Zucker-Bausteinen aufgebaut sind, beispielsweise Xylo- und Arabinofuranose Derivate, ringerweiterte und ringverengte Zucker, acyclische, ringverbrückte oder geeignete andersartige Zucker-Derivate. Die Erfindung ist ferner nicht auf die in Formel I beispielhaft aufgeführten Derivate des Phosphat-Restes beschränkt, sondern bezieht sich auch auf die bekannten Dephospho-Derivate.

Die erfindungsgemäßen Oligonukleotide können also in vielfältiger Weise von der natürlichen Struktur abgewandelt sein. Solche Modifikationen, die nach an sich bekannten Methoden eingeführt werden, sind beispielsweise:

a) Modifikationen der Phosphatbrücke
Beispielhaft seien genannt: Phosphorothioate, Phosphorodithioate, Methylphosphonate, Phosphoramidate, Boranophosphate, Phosphatmethylester, Phosphatethylester, Phenylphosphonate. Bevorzugte Modifikationen der Phosphatbrücke sind Phosphorothioate, Phosphorodithioate und Methylphosphonate.

b) Ersatz der Phosphatbrücke
Beispielhaft seien genannt: Ersatz durch Formacetal, 3'-Thioformacetal, Methylhydroxylamin, Oxim, Methylendimethylhydrazo, Dimethylensulfon, Silylgruppen. Bevorzugte ist der Ersatz durch Formacetale und 3'-Thioformacetale.

c) Modifikationen des Zuckers
Beispielhaft seien genannt: α-anomere Zucker, 2'-O-Methylribose, 2'-O-Butylribose, 2'-O-Allylribose, 2'-Fluoro-2'-desoxyribose, 2'-Amino-2'-desoxyribose, α-Arabinofuranose, Carbocyclische Zuckeranaloge. Bevorzugte Modifikation ist die durch 2'-O-Methylribose und 2'-O-n-Butylribose.

d) Modifikationen des Zuckers und der Phosphat-Brücke
Beispielhaft seien genannt die Peptid-Nukleinsäuren (PNA's), bei denen das Zucker/Phosphat-Rückgrat durch ein Aminoethylglycin-Rückgrat ersetzt ist, sowie die carbamatverbrückten Morpholino-Oligomeren.

e) Andere Modifikationen der Basen, insbesondere der Pyrimidinbasen Beispielhaft seien genannt: 5-Propinyl-2'-desoxyuridin, 5-Propinyl-2'-desoxycytidin, 5-Hexinyl-2'-desoxyuridin, 5-Hexinyl-2'-desoxycytidin, 5-Fluoro-2'-desoxycytidin, 5-Fluor-2'-desoxyuridin, 5-Hydroxymethyl-2'-desoxyuridin, 5-Methyl-2'-desoxycytidin, 5-Brom-2'-desoxycytidin. Bevorzugte Modifikationen sind 5-Propinyl-2'-desoxyuridin, 5-Hexinyl-2'-desoxyuridin, 5-Hexinyl-2'-desoxycytidin und 5-Propinyl-2'-desoxycytidin.

f) 3'-3'- und 5'-5'-Inversionen [z.B. M. Koga et al., J. Org. Chem. 56 (1991) 3757]

g) 5'- und 3'-Konjugate.
Beispielhaft für Gruppen, die die intrazelluläre Aufnahme begünstigen, sind verschiedene lipophile Reste wie $-O-(CH_2)_x-CH_3$, worin x eine ganze Zahl von 6 bis 18 bedeutet, $-O-(CH_2)_n-CH = CH-(CH_2)_m-CH_3$, worin n und m unabhängig voneinander eine ganze Zahl von 6 bis 12 bedeuten, $-O-(CH_2CH_2O)_4-(CH_2)_9-CH_3$, $-O-(CH_2CH_2O)_8-(CH_2)_{13}-CH_3$ und $-O-(CH_2CH_2O)_7-(CH_2)_{15}-CH_3$, aber auch Steroid-Reste wie Cholesteryl oder Vitamin-Reste wie Vitamin E, Vitamin A oder Vitamin D und andere Konjugate, die natürliche Carriersysteme ausnutzen wie Gallensäure, Folsäure, 2-(N-Alkyl, N-Alkoxy)-Aminoanthrachinon und Konjugate der Mannose und Peptide der entsprechenden Rezeptoren, die zur rezeptorvermittelten Endozytose der Oligonucleotide führen wie EGF (Epiderrnal Growth Factor), Bradykinin und PDGF (Platelet Derived Growth Factor). Unter Markierungs-Gruppen sind fluoreszierende Gruppen beispielsweise von Dansyl-=N-Dimethyl-1-aminonaphthyl-5-sulfonyl-), Fluorescein- oder Coumarin-Derivaten oder chemilumineszierende Gruppen beispielsweise von Acridin-Derivaten zu verstehen sowie das über ELISA nachweisbare Digoxygenin-System, die über das Biotin/Avidin-System nachweisbare Biotin-Gruppe oder aber Linker-Arme mit funktionellen Gruppen, die eine nachträgliche Derivatisierung mit nachweisbaren Reporter-Gruppen gestatten, beispielsweise ein Aminoalkyl-Linker, der mit einem Acridinium-Aktivester zur Chemilumineszenz-Probe umgesetzt wird. Typische Markierungsgruppen sind:

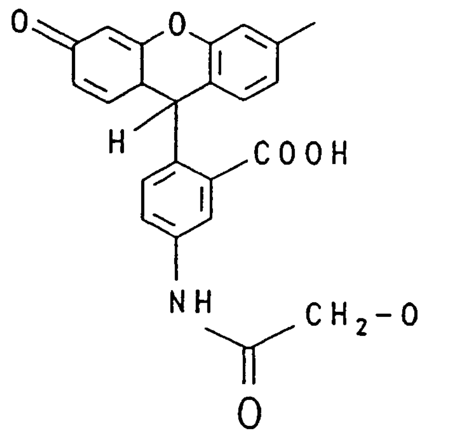

Fluorescein-Derivat

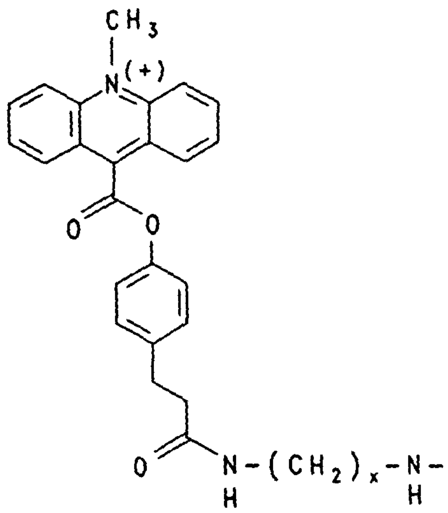

Acridinium-Ester

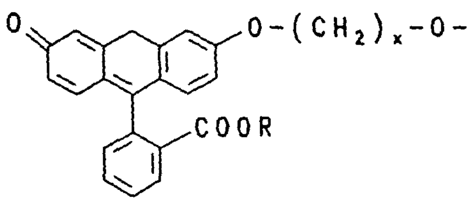

x = 2-18 bevorzugt 4

R = H oder $C_1$-$C_4$-Alkyl

(= "Fluorescein" fuer x = 4 und R = $CH_3$)

Fluorescein-Derivat

R = H oder Aminoschutzgruppe

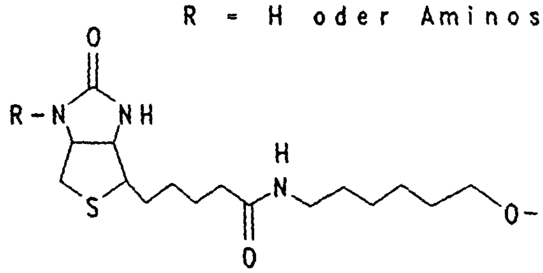

Biotinkonjugat (= "Biotin" fuer R = Fmoc)

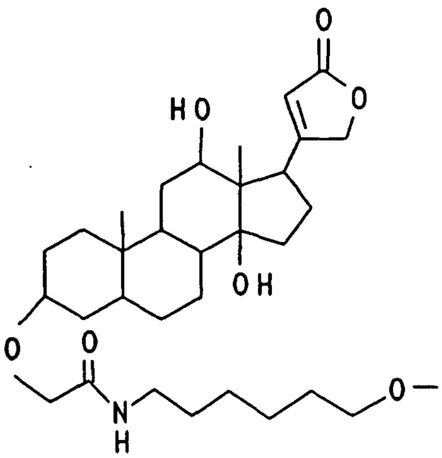

Digoxgeninkonjugat

Oligonucleotidanaloga, die an Nucleinsäuren binden bzw. interkalieren und/oder spalten oder quervernetzen, enthalten z. B. Acridin-, Psoralen-, Phenanthridin, Naphtochinon-, Daunomycin- oder Chlorethylaminoaryl-Konjugate. Typische interkalierende und quervernetzende Reste sind:

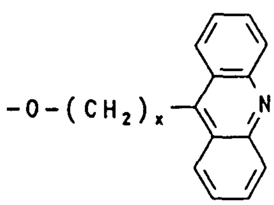

Acridinderivat x = 2-12, bevorzugt 4

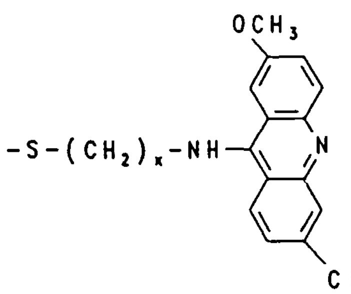

x = 2-12, bevorzugt 4

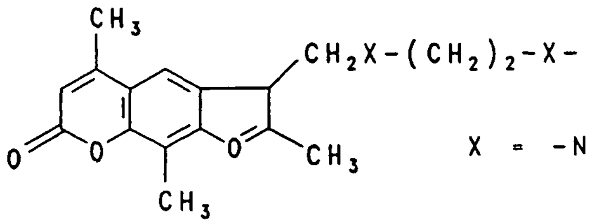

X = -NH oder -O-

Trimethylpsoralen-konjugat (= "Psoralen" fuer X = O)

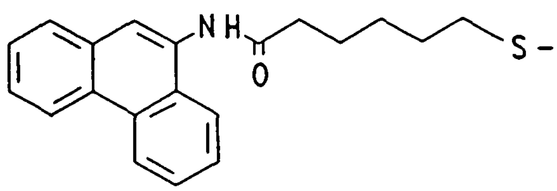

Phenanthrolinkonjugat

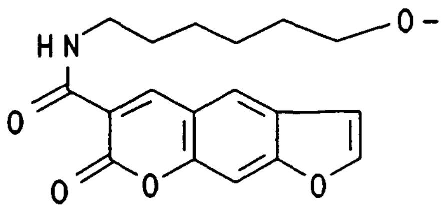

Psoralenkonjugat

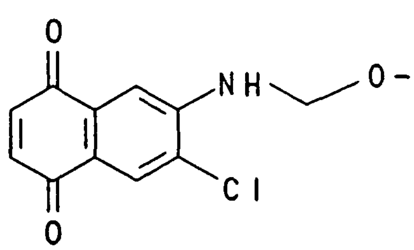

Naphthochinonkonjugat

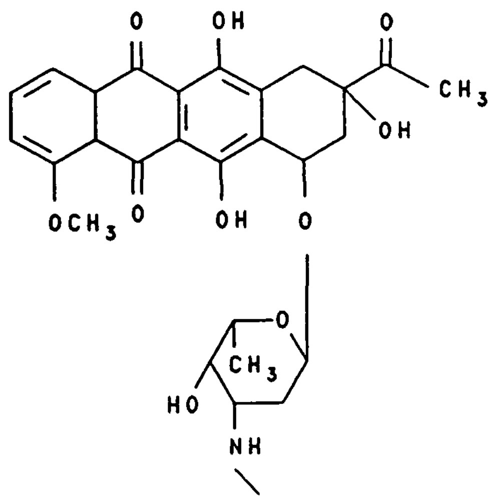

Daunomycinderivat

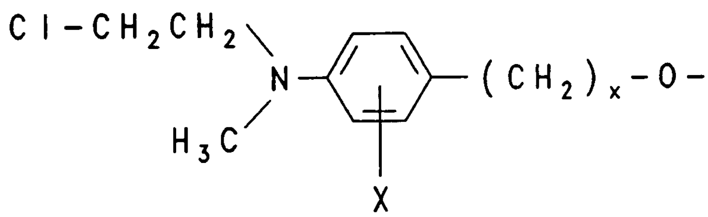

x = 1-18, X = Alkyl, Halogen, $NO_2$, CN, 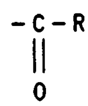

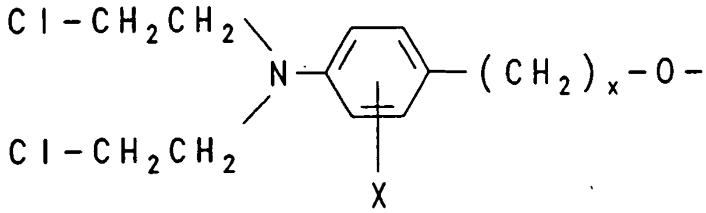

x = 1-18, X = Alkyl, Halogen, $NO_2$, CN, 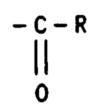

Beispielhaft für Gruppen $NR^3R^4$, in denen $R^3$ und $R^4$ zusammen mit dem sie tragenden Stickstoffatom einen 5- bis 6-gliedrigen heterocyclischen Ring bilden, der zusätzlich ein weiteres Heteroatom enthält, seien der Morpholinyl- und der Imidazolidinyl-Rest genannt.

Gegenstand der Erfindung ist weiterhin die Verbindung der Formel V

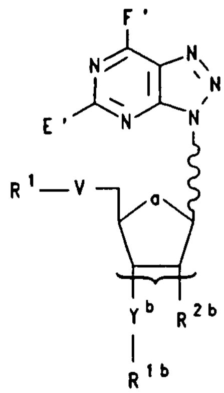

(V)

worin

V Oxy, Sulfandiyl oder Imino ist;
$Y^b$ Oxy, Sulfandiyl, Imino oder Methylen ist;
a für Oxy oder Methylen steht;
$R^{2b}$ Wasserstoff, $OR^{12}$, $C_1$-$C_{18}$-Alkoxy, $C_1$-$C_6$-Alkenyloxy, insbesondere Allyloxy, Halogen, Azido oder $NR^{10}R^{11}$ bedeutet;
$R^1$ eine in der Nukleotidchemie übliche Schutzgruppe bedeutet;
$R^{1b}$ einen Rest der Formel IIc oder IId bedeutet

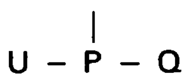 (IIc) 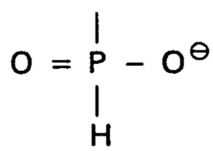 (IId)

worin

U $O$-$R^7$ oder $S$-$R^7$ bedeutet;
Q einen Rest $-NR^8R^9$ bedeutet
$R^7$ $-(CH_2)_2$-CN bedeutet;
$R^8$ und $R^9$ gleich oder verschieden sind und $C_1$-$C_6$-Alkyl, insbesondere Isopropyl oder Ethyl bedeuten oder zusammen mit dem sie tragenden Stickstoffatom einen 5-9-gliedrigen heterozyklischen Ring bedeuten, der zusätzlich ein weiteres Heteroatom aus der Reihe O, S, N enthalten kann, insbesondere

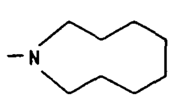 , 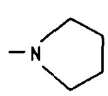 oder 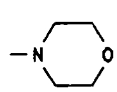 ;

E' und F' unabhängig voneinander H, OH, oder $NR^{10}R^{11}$ bedeutet,

worin

$R^{10}$ und $R^{11}$ gleich oder verschieden sind und Wasserstoff oder eine in der Nukleotidchemie übliche Aminoschutzgruppe bedeuten oder $R^{10}$ und $R^{11}$ gemeinsam eine in der Nukleotidchemie übliche Aminoschutzgrup-

pe bilden,

$R^{12}$ eine in der Nukleotidchemie übliche Hydroxylschutzgruppe, wie beispielsweise t-Butyl-dimethyl-silyl, Tri-isopropyl-silyl, o-nitro-Benzyl, p-nitro-Benzyl, Tris(1-methylethyl)silyl oder 2-Fluorphenyl-4-methoxypiperidin-4-yl (FPMP) bedeutet,

und die geschweifte Klammer andeutet, daß sich $R^2$ und der benachbarte Phosphorylrest in 2'- und 3'-Stellung oder auch umgekehrt in 3'- und 2'-Stellung befinden können.

Eine bevorzugte Ausführungsform stellen Verbindungen der Formel (V) dar, in denen V, $Y^b$ und a für Oxy stehen, $R^{2b}$ Wasserstoff oder $OR^{12}$, insbesondere Wasserstoff und $R^{1b}$ einen Rest der Formel (IIc) oder (IId) bedeutet, wobei U $O-(CH_2)_2-CN$ bedeutet und $R^8$ und $R^9$ gleich oder verschieden sind und Isopropyl oder Ethyl bedeuten. Ganz besonders bevorzugt sind diese, falls sich zusätzlich die Base am Zucker in β-Stellung und $R^{2b}$ in 2'-Stellung befindet.

Gegenstand der Erfindung sind auch Verbindungen der Formel V, in denen die Verknüpfung mit dem Zuckerrest über das N2-Atom der 8-Azapurinbase erfolgt.

Bevorzugte Aminoschutzgruppen sind beispielsweise Acyl- oder AmidinSchutzgruppen.

Die Erfindung betrifft auch Verbindungen der Formel VI

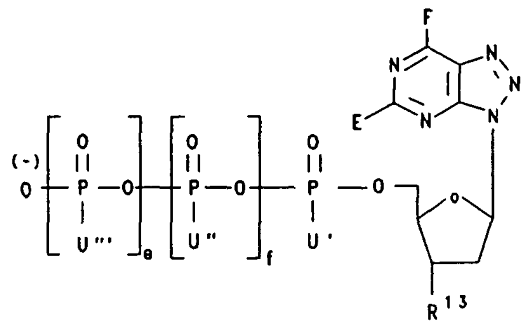

(VI)

worin unabhängig voneinander

U' = U" = U'" gleich Hydroxyl oder Mercapto ist;

e und f 0 oder 1 bedeuten;

$R^{13}$ Wasserstoff oder OH bedeutet und

E und F H, OH oder $NH_2$ bedeuten,

wobei Verbindungen der Formel VI ausgenommen sind, in denen U', U", U'", $R^{13}$ und F OH bedeuten, E gleich $NH_2$ ist und e und f 1 bedeuten.

Bevorzugt sind Verbindungen der Formel VI in denen U' Hydroxyl oder Mercapto bedeutet, U" = U'" Hydroxyl bedeutet und e und/oder f gleich 1 ist.

Bevorzugt sind des weiteren Verbindungen der Formel VI, in denen

E gleich H und F gleich $NH_2$ ist oder falls E gleich $NH_2$ und F gleich OH ist, $R^{13}$ gleich H ist bzw. falls

E gleich $NH_2$ und F, $R^{13}$, U', U" und U'" gleich OH sind, daß e und/oder f gleich 0 ist bzw. falls

E gleich $NH_2$ und F, $R^{13}$, U" und U'" gleich OH und e und f gleich 1 sind, daß U' gleich Mercapto ist.

Die erfindungsgemäßen Verbindungen der Formel VI können als Hilfsmittel in der Molekularbiologie eingesetzt werden, beispielsweise in PCR-Reaktionen (e = f = 1, $R^{13}$ = OH) oder zum Sequenzieren (e = f =1; $R^{13}$ = H oder OH).

Die Darstellung der Verbindungen der Formel VI ist ausgehend von den entsprechenden 8-Azapurin-Nucleosiden möglich und erfolgt nach allgemein bekannten Methoden. Bevorzugt können die Verbindungen der Formel VI nach einem verkürzten Eintopfverfahren von Ludwig in Gegenwart von 1,8-Bis(dimethylamino)naphathalin und Trimethylphosphat hergestellt werden [J. Ludwig et al., (1981) Acta Biochem. Biophys. Sci. Hung., 16, 131].

Die Erfindung umfaßt auch alle tautomeren Formen der Verbindungen der Formel I, V und VI, insbesondere alle tautomeren Formen der 8-Azapurinbasen der Formel IV.

Gegenstand der Erfindung ist weiterhin ein Verfahren zur Herstellung der erfindungsgemäßen Verbindungen der Formel I.

Die Herstellung 8-Azaguanin enthaltender Triribonucleosid-Diphosphate auf enzymatischen bzw. chemisch/enzymatischen Weg ist beschrieben [Grünberger et al., Biochim. Biophys. Acta, (1968) 161, 147-155]. Von Bodnar et al. wird die enzymatische Synthese doppelsträngiger, 8-Azaguanin enthaltender Phagen-DNA mittels DNA-Polymerase beschrieben [Bodnar et al., (1983) J. Biol. Chem., 258, 15206-15213].

Aufgrund der Tatsache, daß die N-Glycosidische Bindung von 8-Azadesoxyguanosin äußerst säurestabil ist [Seela et al., (1993), Helv. Chim. Acta, 76, 2388-2397] können zur Herstellung der erfindungsgemäßen, 8-Azapurin enthaltenden Oligonukleotide die in der chemischen Synthese von Oligonucleotiden üblichen Standardbedingungen eingesetzt werden.

Die Darstellung der erfindungsgemäßen Verbindungen der Formel I erfolgt in Lösung oder vorzugsweise an fester Phase, gegebenenfalls unter Zuhilfenahme eines automatischen Synthesegeräts. Der Aufbau der Oligomere der Formel I kann schrittweise erfolgen, indem sukzessive eine Mononucleotid mit jeweils einer Nucleobase an einen entsprechend derivatisierten Träger oder an eine wachsende Oligomerkette ankondensiert werden.

Der Aufbau des Oligonucleotide erfolgt nach den dem Fachmann bekannten Verfahren wie der Triester-Methode, der H-Phosphonat-Methode oder Phosphoramidit-Methode, [E. Sonveaux, (1986), Bioorganic Chemistry, 14, 274-325; S.L. Beaucage et al., (1992), Tetrathedron, 48, 2223-2311]. Zur Einführung der 8-Azapurinderivate werden bevorzugt die Nukleotid-Monomerbausteine der Formel V, insbesonders bevorzugt die der Formel V in der E' gleich $NR^{10}R^{11}$ und F' gleich OH ist eingesetzt.

Die Bereitstellung der Verbindungen der Formel V als Bausteine für die Oligonucleotid-Festphasensynthese kann ausgehend von den entsprechenden 8-Azapurin-Nucleosiden erfolgen. Nach Einführung geeigneter Schutzgruppen für die Aminogruppen der 8-Azapurinbasen sowie der freien 5'-Hydroxylgruppe des Zuckers werden die Monomere in die entsprechenden Phosphonat- bzw. Phosphoramidit- Derivate übergeführt. Die Einführung geeigneter Aminoschutzgruppen, z.B. in Form einer Formamidin-Schutzgruppe ((Dimethylamino)methyliden) oder Acylschutzgruppe erfolgt nach allgemein bekannten Methoden [L.J. Mc Bride et al, (1983) Tetrahedron Lett., 24, 2953, G.S. Ti et al, (1982) J. Am. Chem. Soc., 104, 1316; H. Schaller et al. (1963), J. Am. Chem. Soc., 85, 3821], wobei im Falle der Acylierung der Aminogruppe die Anwendung der Peracylierungsmethode nach Schaller vorteilhaft ist.
Eine geeignete Schutzgruppe für die freie 5'-OH-Gruppe des Zuckers ist z. B. 4,4'-Dimethoxytrityl, deren Einführung ebenfalls nach bekannten Methoden erfolgt [C.B. Reese (1978), Tetrahedron, 34, 3143; D. Flockerzi et al., (1981), Liebigs Ann. Chem.,1568]. Die solchermaßen geschützten Monomere können entsprechend einer Vorschrift von Froehler et al zu den entsprechenden Phosphonaten umgesetzt werden [B. C. Froehler et al., (1986), Nucl. Acid Res., 14, 5399]. Die Herstellung von Cyanoethyl-Phosphoramidit-Derivaten kann beispielsweise durch Umsetzung der Monomere mit Chlor-β-cyanoethoxy-(N,N-diisopropylamino)phosphan in wasserfreien Dichlormethan erfolgen [N. D. Sinha et al., (1984) Nucl. Acid Res., 12, 4539].

Die Oligoribonucleotidsynthese wird im Vergleich zur Desoxyribooligonucleotidsynthese durch die zusätzliche 2'-OH-Gruppe erschwert. Die erste Schwierigkeit besteht darin, eine Kombination kompatibler 2'- und 5'-OH-Schutzgruppen zu finden. So muß der Rest an der O-2'-Position bei der Oligonucleotidsynthese gegenüber den sauren Bedingungen der Hydrolyse der Tritylschutzgruppen stabil sein. Ferner müssen Bedingungen, die zu einer Wanderung des Phosphatrestes von der 3'- zur 2'-Position führen könnten vermieden werden. Bei der Einführung der Schutzgruppe ist eine regioselektive Reaktion wünschenswert.

Vorteilhaft für die Synthese der erfindungsgemäßen Oligoribonucleotide der Formel (I) ist die Verwendung von Triisopropylsilylchlorid als 2'-OH-Schutzgruppe, mit der hohe Selektivitäten bei ausreichender Stabilität und milden Abspaltungsbedingungen (TBAF/THF) erzielt werden. Die Selektivität der Reaktion kann noch gesteigert werden, wenn statt Imidazol Silbernitrat als Katalysator verwendet wird [F. Seela, K. Mersmann, J.A. Grasby, M.J. Gait, Helv. Chim. Acta 1993, 76, 1809.

Für die Festphasensynthese von Oligoribonucleotiden sind - im Gegensatz zur Festphasensynthese von Desoxyribonukleotiden - Phosphoramiditbausteine weniger geeignet. Das liegt vor allem daran, daß die sterische Hinderung des reaktiven Phosphoramidits durch die sperrige 2'-Silylschutzgruppe relativ lange Kupplungszeiten erfordert [N. Usman, R.T. Pon, K.K. Ogilviee, Tetrahedron Lett. 1985, 26, 4567] und zudem eine geringere Kupplungsausbeute bewirkt. Im Vergleich zu den Phosphoramiditen sind Ribonucleosid-Phosphonate stabil gegenüber Hydrolyse und Oxidation und weisen in der Oligonucleotid-Synthese realtiv kurze Zycluszeiten auf.

Die erfindungsgemäßen Ribonucleotid-Phosphonate können entsprechend einer Vorschrift von Froehler hergestellt werden [B. Froehler, P.G. Nug, M.D. Mateuci, Nucl. Acids Res. 1986, 14, 5399].

Die Synthese von Verbindungen der Formel I, deren Oligonucleotid-Teil am 3'-und/oder am 5'-Ende modifiziert sind, erfolgt bezüglich dieser Modifikationen nach den in EP-A 0 552 766 beschriebenen Verfahren.

Gegenstand der Erfindung sind weiterhin die Verwendung der erfindungsgemäßen Verbindungen der Formel I zur Herstellung eines Arzneimittels sowie ein Verfahren zur Herstellung eines Arzneimittels, das dadurch gekennzeichnet ist, daß man die erfindungsgemäßen Oligonucleotide mit einem physiologisch annehmbaren Träger sowie gegebenenfalls geeigneten Zusatz- und/oder Hilfsstoffen vermischt.

Ganz generell erstreckt sich die vorliegende Erfindung auf die Verwendung von Verbindungen der Formel I

als therapeutisch wirksame Bestandteile eines Arzneimittels. Als therapeutisch wirksame Oligonucleotid-Derivate versteht man im allgemeinen Antisense Oligonucleotide, Tripelhelix-bildende-Oligonucleotide, Aptamere oder Ribozyme, insbesondere Antisense-Oligonucleotide.

Darüberhinaus ist ein weiterer Gegenstand der vorliegenden Erfindung die Verwendung von Oligonukleotiden mit mindestens einem 8-Azapurin, vorzugsweise mit 8-Azaguanin oder 8-Azaadenin als Diagnostikum, beispielsweise zur Detektion der Präsenz oder Absenz oder der Menge eines spezifischen doppelsträngigen oder einzelsträngigen Nucleinsäuremoleküls in einer biologischen Probe.

Die Oligonukleotide haben für die erfindungsgemäße Verwendung eine Länge von 4 bis 100, vorzugsweise von ca. 5-40, insbesondere von ca. 6-30. Nucleotiden. Ansonsten gelten auch hier die oben beschriebenen Vorzugsbereiche, Modifikationen bzw. Konjugationen.

Die Arzneimittel der vorliegenden Erfindung können beispielsweise zur Behandlung von Erkrankungen, die durch Viren hervorgerufen werden, beispielsweise durch HIV, HSV-1, HSV-2, Influenza, VSV, Hepatitis B oder Papilloma Viren, verwendet werden.

Erfindungsgemäße Antisense Oligonucleotide-Derivate, also Antisense Oligonucleotide, in denen mindestens eine Purinbase durch eine 8-Azapurinbase ersetzt ist, und die gegen solche Targets wirksam sind, haben beispielsweise folgende Basensequenz:

a) gegen HIV, z. B.

5′-A C A C C C A A T T C T G A A A A T G G -3′

(I)

oder

5′-A G G T C C C T G T T C G G G C G C C A-3′

(II)

oder

5′-G T C G A C A C C C A A T T C T G A A A A T G G A T A A-3′

(III)

oder

5′-G C T A T G T C G A C A C C C A A T T C T G A A A-3′

(IV)

oder

5'-C T G T C T C C G C T T C T T C T T C C T G C C A T A G G A G

(V)

oder

5'-T C G T C G C T G T C T C C G C T T C T T C T T C C T G C C A

(VI)

oder

b) gegen HSV-1, z.B.

5'-G C G G G G C T C C A T G G G G G T C G-3'

(VII)

Die Arzneimittel der vorliegenden Erfindung eignen sich beispielsweise auch zur Behandlung von Krebs. Beispielsweise können dabei Oligonucleotid-Sequenzen zum Einsatz kommen, die gegen Targets gerichtet sind, die für Krebsentstehung bzw. Krebswachstum verantwortlich sind. Solche Targets sind beispielsweise:

1) Nucleare Onkoproteine wie beispielsweise c-myc, N-myc, c-myb, c-fos, c-fos/jun, PCNA, p120

2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl

3) Zelluläre Rezeptoren wie beispielsweise EGF-Rezeptor, c-erbA, Retinoid-Rezeptoren, Protein-Kinase regulatorische Untereinheit, c-fms

4) Cytokine, Wachstumsfaktoren, Extrazelluläre Matrix wie beispielsweise CSF-1, IL-6, IL-1a, IL-1b, IL-2, IL-4, bFGF, Myeloblastin, Fibronectin,

Erfindungsgemäße Antisense-Oligonucleotide der Formel I, die gegen solche Targets wirksam sind, haben beispielsweise folgende Basen-Sequenz:

a) gegen c-Ha-ras , z. B.

5'- C A G C T G C A A C C C A G C -3'

(VIII)

c) c-myc, z.B.

5'- G G C T G C T G G A G C G G G G C A C A C-3'

(IX)

5'-A A C G T T G A G G G G C A T-3'
(X)

d) c-myb, z.B.

5'-G T G C C G G G G T C T T C G G G C -3'
(XI)

e) c-fos, z.B.

5'-G G A G A A C A T C A T G G T C G A A A G-3'
(XII)

5'-C C C G A G A A C A T C A T G G T C G A A G-3'
(XIII)

5'-G G G G A A A G C C C G G C A A G G G G-3'
(XIV)

f) p120, z.B.

5'-C A C C C G C C T T G G C C T C C C A C-3'
(XV)

g) EGF-Rezeptor, z.B.

5'-G G G A C T C C G G C G C A G C G C -3'
(XVI)

5'-G G C A A A C T T T C T T T T C C T C C-3'

(XVII)

h) p53 Tumorsuppressor, z.B.

5'- G G G A A G G A G G A G G A T G A G G-3'

(XVIII)

5'-G G C A G T C A T C C A G C T T C G G A G-3'r

(XIX)

Die Arzneimittel der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM, VCAM oder ELAM.

Erfindungsgemäße Antisense-Oligonucleotid-Derivate, die gegen solche Targets wirksam sind, haben beispielsweise folgende Basen-Sequenz:

a) VLA-4, z.B.

5'-G C A G T A A G C A T C C A T A T C -3'

(XX)

oder

b) ICAM, z.B.

5'- C C C C C A C C A C T T C C C C T C T C-3'

(XXI)

5'-C T C C C C C A C C A C T T C C C C T C-3'

(XXII)

5'-G C T G G G A G C C A T A G C G A G G-3'

(XXIII)

c) ELAM-1, z. B.

5'-A C T G C T G C C T C T T G T C T C A G G -3'
(XXIV)

5'- C A A T C A A T G A C T T C A A G A G T T C-3'
(XXV)

Die Arzneimittel der vorliegenden Erfindung eignen sich beispielsweise auch zur Verhinderung der Restenose. Beispielsweise können dabei Oligonucleotid-Sequenzen zum Einsatz kommen, die gegen Targets gerichtet sind, die für Proliferation oder Migration verantwortlich sind. Solche Targets sind beispielsweise:

1) Nucleare Transaktivator-Proteine und Cycline wie beispielsweise c-myc, c-myb, c-fos, c-fos/jun, Cycline und cdc2-Kinase

2) Mitogene oder Wachstumsfaktoren wie beispielsweise PDGF, bFGF, EGF, HB-EGF und TGF-β.

3) Zelluläre Rezeptoren wie beispielsweise bFGF-Rezeptor, EGF-Rezeptor und PDGF-Rezeptor.

Erfindungsgemäße Oligonucleotide der Formel I, die gegen solche Targets wirksam sind, haben beispielsweise folgende Basen-Sequenz:

a) c-myb

5'-G T G T C G G G G T C T C C G G G C-3'
(XXVI)

b) c-myc

5'-C A C G T T G A G G G G C A T-3'
(XXVII)

c) cdc2-Kinase

5'- G T C T T C C A T A G T T A C T C A-3'
(XXVIII)

d) PCNA (proliferating cell nuclear antigen of rat)

5'- G A T C A G G C G T G C C T C A A A-3'

(XXIX)

Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Der Einschluß der Arzneimittel in Liposomen, die gegebenenfalls weitere Komponenten wie Proteine enthalten, ist eine ebenfalls geeignete Applikationsform. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

Bevorzugte Verabreichungsformen sind topische Applikationen, lokale Applikationen wie beispielsweise mit Hilfe eines Katheters oder aber auch Injektionen. Zur Injektion werden die Antisense-Oligonucleotid-derivate in einer flüssigen Lösung, vorzugsweise in einem physiologisch annehmbaren Puffer, wie z.B. Hank's Lösung oder Ringer's Lösung, formuliert. Die Antisense-Oligonucleotide können aber auch in fester Form formuliert werden und vor dem Gebrauch gelöst oder suspendiert werden. Die für die systematische Verabreichung bevorzugten Dosierungen betragen ca. 0,01 mg/kg bis ca. 50 mg/kg Körpergewicht und Tag.

Ganz generell erstreckt sich die Erfindung auf die Verwendung von Verbindungen der Formel I als DNA-Sonden oder Primer in der DNA-Diagnostik und allgemein als Hilfsmittel in der Molekularbiologie

Beispiele:

Die in den Beispielen genannten Verbindungen (1) - (16) weisen folgende Strukturformel auf.

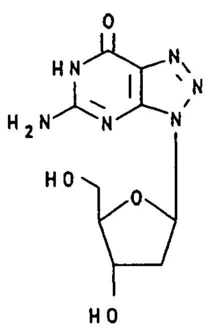

1

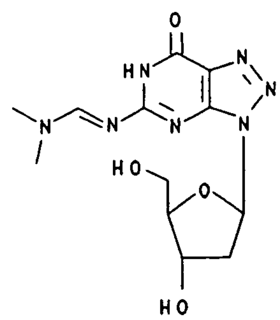

2

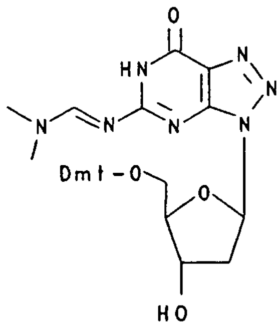

3

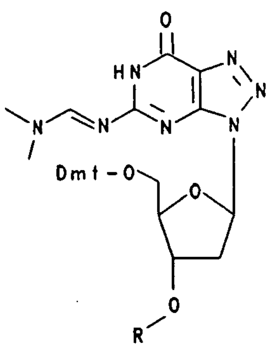

| | R |
| --- | --- |
| 4 | 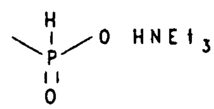 |
| 5 | 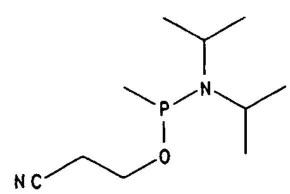 |
| 6 | 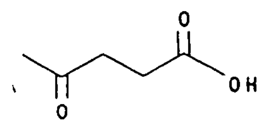 |

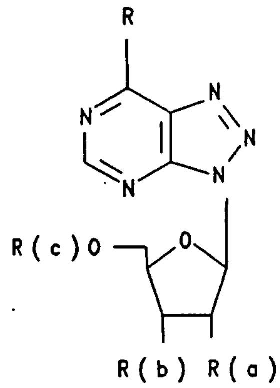

| | R(a) | R(b) | R(c) | R |
|---|---|---|---|---|
| (7) | $-COCH_3$ | $-COCH_3$ | $-COCH_3$ | $-NH-CO-C_5H_{19}$ |
| (8) | $-COCH_3$ | $-COCH_3$ | $-COCH_3$ | $NH_2$ |
| (9) | OH | OH | OH | $NH_2$ |
| (10) | OH | OH | OH | $-NH-CO-C_6H_5$ |
| (11) | OH | OH | OH | $-N = CH-N(CH_3)_2$ |
| (12) | OH | OH | OH | $CH_3$ $-N = CH-N(CH_3)_2$ |
| (13) | OH | OH | Dmt | " |
| (14) | Tms | OH | Dmt | " |
| (15) | OH | Tms | Dmt | " |
| (16) | Tms | TEP | Dmt | " |

Beispiel 1:

5-Amino-3-(2-desoxy-β-D-erythro-pentofuranosyl)-3H-1,2,3,-triazolo[4,5-d]pyrimidin-7-(6H)-on- 5'-O-Triphosphat, Triethylammoniumsalz (8-Aza-2'-desoxyguanosin 5'-Triphosphat)

3-(2-Desoxy-β-D-erythro-pentofuranosyl)-5-amino-3H-1,2,3,-triazolo[4,5d] pyrimidin-7-(6H)-on (8-Aza-2'-desoxyguanosin (1)) (26 mg; 0,09 mmol) wurde zusammen mit 1.8-Bis(dimethylamino)naphthalin (33 mg, 0,15 mmol) in Trimethylphosphat (0,25 ml) unter leichten Erwärmen in Lösung gebracht. Nach Kühlen auf 0°C erfolgte eine Zugabe von frisch destilierten $POCl_3$ (12 µl, 0,13 mmol). Die Reaktion wurde für 4 h bei 4° C gehalten und anschließend eine Lösung aus Tri-n-butylammoniumdiphosphat (0,5 m in DMF, 1 ml) und Tri-n-butylamin (100 µl, 0,42 mmol) zugegeben. Nach 3 min Rühren bei 0°C wurde die Mischung mit 1 M TBK-Puffer (10 ml) versetzt und zur Trockene eingedampft. Der Rückstand wurde an DEAD Sephadex® (Säule 1,5 x 20 cm, $HCO_3$ -Form) chromatographiert. Nach Waschen mit ca. 500 ml $H_2O$ wurde mit einem linearen Gradienten von $H_2O$/0,9 M TBK-Puffer (je 1 l) chromatographiert. Dabei erhielt man eine Hauptzone bei ca. 0,5 M TBK-Puffer (0,019 mM, 20%). DC (Kieselgel, i-Propanol/$H_2O$/$NH_3$, 3:1:1): $R_f$ 0,2. UV ($H_2O$): $\lambda_{max}$ 256 nm. $^{31}$P-NMR (0,1 M Tris-HCl, pH 8,0, 100 mM EDTA/$D_2O$):-10,27 (d,J = 19,3, $P_\chi$ ); -10,63 (td, J = 20,2 und 6,0, $P_\alpha$ ); -22,60 (t, J = 19,8, $P_\beta$ ).

Beispiel 2:

3-(2-Desoxy-β-D-erythro-pentofuranosyl)-5-{[(dimethylamino)methyliden] amino}-3H-1,2,3,-triazolo[4,5d] pyrimidin-7-(6H)-on (2).

3-(2-Desoxy-β-D-erythro-pentofuranosyl)-5-amino-3H-1,2,3,-triazolo[4,5d] pyrimidin-7-(6H)-on (8-Aza-2'-desoxyguanosin (1)) (290 mg; 1,06 mmol) wurden in abs. DMF (7 ml) gelöst und mit N,N-Dimethylformamiddiethylacetal (5 ml) versetzt. Nach 24 Stunden Rühren bei Raumtemperatur wurde der Ansatz im Vakuum einrotiert und mit Toluol koevaporiert. Die Reinigung der Rückstandes erfolgte mittels Chromatographie an Kieselgel (Säule: 20 x 4 cm, 0.5

bar, $CH_2Cl_2$/MeOH 8:2). Man erhielt Verbindung (2) (320 mg, 92%) als farblosen Schaum, der aus MeOH kristallisierte. Smp. 236°C.
DC ($CH_2Cl_2$/MeOH 8:2):$R_f$ 0.7. UV (MeOH): 301 (26500), 244(15500).
$^1$H-NMR (($D_6$)DMSO: 8.66 (s, CH); 6.43 (t, J = 6.32, H-C(1')); 5.40 (br.s, OH-C(3')); 4.79 (br.s, OH-C(5')); 4.48 (m, H-C (3')); 3.85 (m, H-C(4')); 3.57 (m, H-C(5')); 3.19, 3.06 (2 s, $2CH_3$); 2.90 (m, $H_\beta$-C(2')); 2.34 (m, $H_{(\alpha)}$-C(2')).

| | |
|---|---|
| $C_{12}H_{17}N_7O_4$ (323.31) | Ber. C 44.56, H 5.29. N 30.32 |
| | Gef. C 44.64, H 5.26, N 30.37. |

Beispiel 3:

3-[2-Desoxy-5-O-(4,4'-dimethoxytrityl-β-D-erythro-pentofuranosyl] 5{[(dimethylamino)methyliden]-amino}-3H-1,2,3-triazolo[4,5-d]pyrimidin-7-(6H)-on (3).

Das aminogeschützte 8-Aza-2'-desoxyguanosin (2) aus Beispiel 2 (170 mg, 0.53 mmol) wurde durch wiederholtes Abdampfen mit abs. Pyridin behandelt und anschließend in 6 ml des selbigen aufgenommen. Bei Raumtemperatur wurde 4,4'Dimethoxytritylchlorid (260 mg, 0.7 mmol) hinzugegeben und 3 Stunden gerührt. Die Lösung wurde dann in 5% $NaHCO_3$ (40 ml) gegossen und zweimal mit je 30 ml $CH_2Cl_2$ extrahiert. Die vereinigten organischen Phasen wurden über $Na_2SO_4$ getrocknet und im Vakuum einrotiert. Der Rückstand wurde an Kieselgel chromatographiert (Säule: 15 x 4 cm, 0.5 bar, $CH_2Cl_2$/MeOH 95:5). Die Ausbeute betrug 270 mg (82%), farbloser Schaum. DC ($CH_2Cl_2$/MeOH 95:5) $R_f$ 0.3. UV (MeOH): 302 (24600), 235 (35000). $^1$H-NMR (($D_6$)DMSO): 12.03 (s, NH); 8.66 (s, CH); 7.39 - 6.70 (2 m, aromat. H); 6.48 (m, H-C(1')); 4.57 (m, H-C(3')); 3.97 (m, H-C(4')); 3.69 (s, 2 $OCH_3$); 3.37 (m, H-C(5')), 3.22, 3.07 (2s, $2CH_3$); 2.90 (m, $H_\beta$-C(2')); 2.40 (m, $H_{(\alpha)}$-C(2')).

| | |
|---|---|
| $C_{33}H_{35}N_7O_6$ (625.68) | Ber. C 63.34, H 5.64, N 15.67 |
| | Gef. C 63.42, H 5.72, N 15.71 |

Beispiel 4:

3-[2-Desoxy-5-O-(4,4'-dimethoxytrityl)-β-D-erythro-pentofuranosyl] 5-{[(dimethylamino)methyliden]-amino}-3H-1,2,3-triazolo[4,5-d]pyrimidin-7-(6H)-on 3'(Triethylammonium Phosphonat) (4).

Zu einer Lösung von $PCl_3$ (200 µl, 0.22 mmol) sowie N-Methylmorpholin (2.7 ml, 2.24 mmol) in $CH_2Cl_2$ (10 ml) wurde 1,2,4-Triazol (0.54 g, 7.56 mmol) gegeben. Die Lösung ließ man 30 min rühren und dann auf 0°C abgekühlen. Die 5'-O-Dmimethoxytrityl-Verbindung 3 aus Beispiel 3 (220 mg, 0.35 mmol) wurde durch Abdampfen mit getrocknetem MeCN getrocknet und dann, gelöst in $CH_2Cl_2$ (5 ml), hinzugegeben. Nach 10 min Rühren goß man die Mischung in 1 M ($Et_3NH$)$HCO_3$ (TBK-Puffer, pH 8.0, 30 ml), schüttelte zweimal mit $CH_2Cl_2$ aus und trennte die Phasen. Die vereinigten organischen Lösungen wurden über $Na_2SO_4$ getrocknet und eingedampft. Chromatographie erfolgte an Kieselgel (Säule: 4 x 15 cm, 0.5 bar, 1. $CH_2Cl_2$/$Et_3N$ 98:2; 2. $CH_2Cl_2$/MeOH/$Et_3N$ 88:10:2). Die Substanz der Hauptzone wurde in $CH_2Cl_2$ (10 ml) aufgenommen und mehrere Male mit 0.1 M TBK-Puffer (pH 8.0) ausgeschüttelt. Man erhielt das H-Phosphonat 4 (240 mg, 85%) als farblosen Schaum. DC ($CH_2Cl_2$/MeOH/$Et_3N$ 88:10:2): $R_f$ 0.3. UV (MeOH): 285 (sh, 14900), 303 (18400).
$^1$H-NMR (($D_6$)DMSO): 11.68 (s, NH); 8.83 (s, CH); 7.77, 5.45, (d, J = 585, HP); 7.24-6.69 (2 m, aromat. H); 6.53 (m, J = 4.5, H-C1')); 5.22 (m, H-C(3')); 4.09 (m, H-C(4')); 3.67 (s, 2 $OCH_3$); 3.07, 3.23 (s, 2 $CH_3$, H-C(5')); 2.97 (m, $CH_3\mathit{CH_2}$); 2.56 (m, H-C(2')); 1.13 (m, $\mathit{CH_3}CH_2$).
$^{31}$P-NMR (($D_6$)DMSO): 1.16 ($^1$J (P,H) = 585); $^3$J(P,H-C(3') = 8.90.

| | |
|---|---|
| $C_{39}H_{51}N_8O_8P_1$ (790.87) | Ber. C 59.23, H 6.49, N 14.17 |
| | Gef. C 59.33, H 6.79, N 13.95 |

Beispiel 5:

3-[2-Desoxy-5'-O-(4,4'-dimethoxytrityl)-β-D-erythro-pentofuranosyl] 5-{[(dimethylamino)methyliden]-amino}-3H-1,2,3-triazolo[4,5-d]pyrimidin-7-(6H)-on 3'[(2-Cyanoethyl) N,N-Diisopropylphosphoramidit (5).

Zu einer Lösung von Verbindung (3) aus Beispiel 3 (50 mg, 0.08 mmol) in $CH_2Cl_2$ (1 ml) wurde (i-Pr)$_2$EtN (56 µl, 0.27 mmol) sowie Chloro(2-cyanoethoxy)(diisopropylamino)-phosphan (115 µl, 0.51 mmol) gegeben. Der Ansatz wurde 2 Stunden unter Argon bei Raumtemperatur gerührt. Anschließend wurde die Mischung in 5% $NaHCO_3$ (3 ml)

gegossen und zweimal mit $CH_2Cl_2$ (30 ml) extrahiert. Die vereinigten organischen Phasen wurden über $Na_2SO_4$ getrocknet und einrotiert bis zur Trockene. Die Chromatographie erfolgte an Kieselgel (Säule: 7 x 2 cm, 0.5 bar, $CH_2Cl_2$/AcOEt/$Et_3N$ 45:45:10). Es waren zwei überlappende Zonen von Diastereomeren des Phosphoramidits (5) zu erkennen, welche als farbloser Schaum (35 mg, 55%) erhältlich waren.
DC ($CH_2Cl_2$/AcOEt/$Et_3N$ 45:45:10): $R_f$ 0.4.
$^{31}$P-NMR (($D_6$)DMSO): 149.4, 148.9.

Beispiel 6:

2-[2-Desoxy-5-O-(4,4-dimethoxytrityl)-3'-O-succinyl-β-d-erythro - pentofuranosyl]-5-{[(dimethylamino)methyliden]amino}-3H-1,2,3-triazolo[4,5-d]-pyrimidin-7-(6H)-on (6).

Zu einer Lösung des geschützten Nucleosids (3) aus Beispiel 3 (110 mg, 0.18 mmol) in Pyridin (5 ml) wurde 4-Dimethyl-aminopyridin (30 mg, 0.23 mmol) sowie Bernsteinsäureanhydrid (90 mg, 0.88 mmol) gegeben. Den Ansatz ließ man 48 Stunden bei Raumtemperatur rühren. Die Reaktion wurde durch Zugabe von 2 ml Wasser gestoppt. Nach Eindampfen bis zur Trockene koevaporierte man mit Toluol um restliches Pyridin zu entfernen. Der Rückstand wurde in wenig $CH_2Cl_2$ gelöst und mit einer 10% wässerigen Citronensäurelösung sowie Wasser gewaschen. Die organische Phase wurde über $Na_2SO_4$ getrocknet und im Vakuum eingeengt. Nach Lösen in $CH_2Cl_2$/Pyridin (95:5, 2 ml) gab man schnell n-Pentan/Ether (1:1, 30 ml) hinzu. Der Überstand wurde abfiltriert und es blieb ein farbloses Puder zurück (85 mg, 66%). DC ($CH_2Cl_2$/MeOH 9:1): $R_f$ 0.25.
$^1$H-NMR (($D_6$)DMSO): 8.71 (s, CH); 7.28-6.75 (2 m, aromat. H); 6.46 (m, J = 4.1, H-C(1')); 5.48 (m, H-C(3')); 4.21 (m, H-C(4')); 3.36 (s, 2 $OCH_3$); 3.18, 3.05 (2 s, 2 $CH_3$); 3.10 (m, H-C(5')); 2.51 (m, H-C(2'), 2 $CH_2$).

Beispiel 7

7-(Benzoylamino)-1,2,3-triazolo[4,5-d]pyrimidin

8-Azaadenin (200 mg, 1,47 mmol) wird dreimal in trockenem Pyridin abgedampft, anschließend in 5 ml trokkenem Pyridin aufgenommen. Dann wird Benzoylchlorid (0.28 ml, 2.20 mmol) zugetropft und 3 Stunden bei 60°C gerührt. Danach wird noch eine Stunde unter Rückfluß gekocht. Man läßt die Reaktionsmischung über Nacht stehen und engt auf ca. 1 ml ein. Zu diesem Gemisch gibt man 15 ml kaltes Wasser, läßt 5 min rühren und saugt den entstandenen Niederschlag ab. Der schwach gelbliche Niederschlag wird jeweils zweimal mit 1 ml kaltem Wasser und 1 ml kaltem Acetonitril gewaschen. Man erhält 0.30 g (85 %) farblose Kristalle (MeOH). $F_p$ = 263°C (Zersetzung).
DC (Kieselgel, $CH_2Cl_2$/MeOH = 8:2); $R_f$ = 0.6.
UV (Methanol) $\lambda_{max}$ (ε) = 242 (12100); 291 (16000).
$^1$H-NMR ($D_6$-DMSO) δ: 7.58; 7.69; 8.13 (arom.-$H_5$) 8.89 (s, H-5), 11.99 (s-$N^6$-H).

| | |
|---|---|
| $C_{11}H_8N_6O$ | Ber. C 54.99 H 3.36 N 34.99. |
| | Gef. C 55.10 H 3.34 N 35.04. |

Beispiel 8

7-(Nonanoylamino)-3-[(2,3,5-tri-O-acetyl)-β-D-ribofuranosyl]-3H-1,2,3-triazolo-[4,5-d]pyrimidin (7)

500 mg (1.81 mmol) 7-Nonanoylamido-1,2,3-triazolo[4,5-d]pyrimidin werden zur Trocknung dreimal in trokkenem Pyridin abgedampft. Der Rückstand wird in 20 ml trocknem Acetonitril aufgenommen. 0.58 g (1.81 mmol) 1,2,3,5-Tetra-O-acetyl-β-D-ribofuranose zugefügt, 0.64 ml (5.43 mmol) Zinntetrachlorid zugetropft und die Reaktionsmischung 24 Stunden bei Raumtemperatur gerührt. Die Reaktionslösung wird anschließend auf 25 ml gesättigte Natriumhydrogencarbonatlösung gegeben und viermal mit je 15 ml Dichlormethan extrahiert. Nach dem Trocknen über Natriumsulfat werden die vereinten organischen Phasen zur Trockene eingedampft. Man erhält 0.81 g öligen rückstand. Die Trennung des Produktgemisches erfolgt über Säulenchromatographie (Säule 5.5x30 cm, Kieselgel, Laufmittel: $CH_2Cl_2$/MeOH 95:5). Aus der schneller laufenden Zone erhält man 0.27 g (28 %) farbloses Nucelosid.
DC (Kieselgel, $CH_2Cl_2$/MeOH); $R_f$ = 0.75.
UV (Methanol) $\lambda_{max}$ (ε) = 275 (16800).
$^1$H-NMR ($D_6$-DMSO) δ: 0.82 (m, -$CH_3$-9"); 1.22 (m, -$(CH_2)_5$-); 1.62 (m, J= 6,8 Hz, -$CH_2$-3"); 1.93; 2.08; 2.11 (3s, O = $CCH_3$); 2.61 (t, J= 7.2 Hz, -$CH_2$-2"); 4.26 (m, H-5'); 4.52 (m, H-4'); 5.77 (m, H-3'); 6.12 (m, H-2'); 6.63 (d, J= 3.4 Hz, H-1'); 8.89 (s, H-5); 11.48 (s, br, $N^6$-H).

Beispiel 9

Glycosylierung von 7-Amino-1,2,3-triazolo[4,5-d]pyrimidin mit 1,2,3,5-Tetra-O-acetyl-β-D-ribofuranose.

340 mg (2.5 mmol) 7-Amino-1,2,3-triazolo[4,5-d]pyrimidin und 800 mg (2.5 mmol) 1,2,3,5-Tetra-O-acetyl-β-D-ribofuranose werden in 10 ml trockenem Acetonitril suspendiert. Zu dieser Mischung werden unter Argon 0.88 ml (7.5 mmol) Zinntetrachlorid innerhalb von 5 min zugetropft und 24 Stunden bei Raumtemperatur gerührt. Die entstandene Lösung wird vorsichtig auf 32 ml gesättigte $NaHCO_3$-Lösung gegossen. Der ausgefallene Niederschlag wird abgesaugt und zweimal mit je 10 ml Wasser gewaschen. Filtrat und Waschwasser werden viermal mit je 15 ml Methylenchlorid extrahiert. Nach Trocknung über $Na_2SO_4$ und Abziehen des Lösungsmittels erhält man 0.87 g schwach gelblichen Schaum. Die Trennung der Reaktionsprodukte erfolgt über Säulenchromatographie (Säule: 5.5 x 20 cm, Kieselgel, $CH_2Cl_2$/MeOH 98:2-90:10).

Beispiel 10

7-Amino-3-[(2,3,5-tri-O-acetyl)-β-D-ribofuranosyl]-3H-1,2,3-triazolo[4,5-d]-pyrimidin (8)

Die schneller laufende Zone ergibt 0.34 g (34 %) farblosen Schaum.
DC (Kieselgel, $CH_2Cl_2$/MeOH 9:1): $R_f$ = 0.45
UV (Methanol) $\lambda_{max}$ (ε) = 280 (10900).
$^1$H-NMR ($D_6$-DMSO) δ: 1.89; 2.10; 2.11 (3s, 2',3',5'-O-C = O); 4.20 (m, $H_2$-5'); 4.48 (m, H-4'); 5.74 (t, J = 5.5 Hz, H-3'); 6.07 (t, J = 3.7 Hz, H-2'); 6.48 (d, J = 2.8, H-1'); 8.25; 8.6 (2s, $N^6$-$H_2$), 8.34 (s, H-5).

| | |
|---|---|
| $C_{15}H_{18}N_6O_7$ | Ber. C 45.68 H 4.61 N 21.31. |
| | Gef. C 45.98 H 4.72 N 21.40. |

Beispiel 11

7-Amino-2-[(2,3,5-tri-O-acetyl)-β-D-ribofuranosyl]-2H-1,2,3-triazolo[4,5-d]-pyrimidin

Aus der langsamer laufenden Zone der chromatographischen Aufarbeitung erhält man 0.46 g (47 %) farblosen Schaum.
DC (Kieselgel, $CH_2Cl_2$/MeOH): $R_f$ = 0.35
UV (Methanol) $\lambda_{max}$ (ε) = 253 (3900), 300 (10400).
$^1$H-NMR ($D_6$-DMSO) δ: 1.94; 2.07; 2.11 (3s, 2',3',5'-O-C = O); 4.25 (m, $H_2$-5'); 4.52 (m, H-4'); 5.74 (t, J = 6.0, H-3'); 5.91 (d, J = 3.4, H-2'); 6.53 (s, H-1'); 8.3; 8.45 (2s, $N^6$-$H_2$); 8.34 (H-5).

| | |
|---|---|
| $C_{15}H_{18}N_6O_7$ | Ber. C 45.68 H 4.61 N 21.31. |
| | Gef. C 45.86 H 4.71 N 21.22. |

Beispiel 12

7-Amino-3-(β-D-ribofuranosyl]-3H-1,2,3-triazolo[4,5-d]-pyrimidin (8-Azaadenosin, 9)

2.04 g (5.17 mmol) der Verbindung (8) werden in 5 ml Methanol und 5 ml wäßrigem Ammoniak (25 %) zwei Stunden bei Raumtemperatur gerührt. Nach dem Eindampfen zur Trockene und dem Umkristallisieren in 3 ml Wasser erhält man 1.04 g (75 %) farblose Kristalle, die sich bei 217°C zersetzen
DC (Kieselgel, $CH_2Cl_2$/MeOH = 8:2)): $R_f$ = 0.45.
UV: $\lambda_{max}$ (pH 7) = 279 (11600); $\lambda_{max}$ (pH 1) = 263, $\lambda_{max}$ (pH 14) = 279.
$^1$H-NMR ($D_6$-DMSO) δ: 3.56 (m, $H_2$-5'); 4.01 (m, H-4'), 4.29 (m, 3'); 4.85 (m, H-2'); 5.01 (t, J = 5.9 Hz, HO-5'); 5.28 (d, J = 5.7, HO-3'); 5.56 (d, J = 6.0 HO-2'); 6.15 (d, J = 5.2 Hz, H-1'); 8.31 (s, H-5); 8.53; 8.19 (2s, $N^7H_2$).

Beispiel 13

7-Amino-2-(β-D-ribofuranosyl]-2H-1,2,3-triazolo[4,5-d]-pyrimidin

0.81 g (2.05 mmol) der Verbindung aus Beispiel11 werden in 5 ml Methanol und 5 ml wäßrigen Ammoniak (25 %) zwei Stunden bei Raumtemperatur gerührt. Nach dem Eindampfen zur Trockene und dem Umkristallisieren in 2.5 ml Wasser erhält man 0.32 g (59 %) farblose Kristalle, die sich bei 209°C zersetzen.

DC ($CH_2Cl_2$/MeOH = 8:2): $R_f$ = 0.20
UV (Methanol) $\lambda_{max}$ ($\varepsilon$) = 255 (4400), 263 (4100), 297 (10400).
$^1$H-NMR ($D_6$-DMSO) $\delta$: 3.59 (m, $H_2$-5'); 4.06 (m, H-4'); 4.33 (m, H-3'); 4.61 (m, H-2'); 4.76 (t, J = 5.3 Hz, HO-5'); 5.27 (d, J = 5.8 Hz, HO-3'); 5.68 (d, J = 5.5 Hz, HO-2'); 6.08 (s, J = 3.4 Hz, H-1'); 8.31 (s, H-5); 8.12 (s, $N^7$-$H_2$).

Beispiel 14

7-Benzoylamino-3-(β-D-ribofuranosyl)-3H-1,2,3-triazolo-[4,5-d]pyrimidin (10).

100 mg (0.37 mmol) 7-Amino-3-β-D-ribofuranosyl-3H-1,2,3-triazolo[4,5d]pyrimidin werden in 5 ml trockenem Pyridin vorgelegt. Zu dieser Lösung tropft man unter Argonatmosphäre 0.47 ml (3.7 mmol) Trimethylsilylchlorid. Nach einer halben Stunde Rühren bei Raumtemperatur (DC-Kontrolle) werden 0.25 ml (2.0 mmol) Benzoylchlorid zugetropft und vier Stunden bei Raumtemperatur gerührt. Die Reaktionsmischung wird auf 0-5°C abgekühlt, mit 1 ml Wasser versetzt, nach 5 min mit 2 ml wäßrigem Ammoniak (25 %) versetzt und nochmals 30 min gerührt. Das Lösungsmittel wird abgezogen und mit Toluol nachgedampft. Der Rückstand wird in 15 ml gesättigter $NaHCO_3$-Lösung aufgenommen, einmal mit 25 ml Methylenchlorid und mehrmals mit Essigsäureethylester extrahiert. Die vereinigten organischen Phasen werden mit $Na_2SO_4$ getrocknet.
Nach dem Abziehen des Lösungsmittels erhält man 0.05 g (36 %) einer farblosen, kristallinen Substanz, die nach Kristallisation aus Methanol bei 188°C unter Zersetzung schmilzt.
DC (Kieselgel, $CH_2Cl_2$/MeOH = 9:1): $R_f$ = 0.25
UV (Methanol) $\lambda_{max}$ ($\varepsilon$) = 242 (9400), 282 (20300).
$^1$H-NMR ($D_6$-DMSO) $\delta$: 3.56 (m, $H_2$-5'); 4.04 (m, H-4'); 4.36 (m, H-3'); 4.84 (t, HO-5'); 4.92 (m, H-2'); 5.33 (d, J = 5.2 Hz, HO-3'); 5.66 (d, J = 5.4 Hz, HO-2'); 6.30 (d, J = 4.3 Hz, 1'); 7.54-8.11 (m, arom.-$H_5$); 8.94 (s, H-5); 11.99 (s, br, $N^6$-H).

| | |
|---|---|
| $C_{16}H_{16}N_6O_6$ | Ber. C 51.60 H 4.34 N 22.57. |
| | Gef. C 51.49 H 4.43 N 22.74. |

Beispiel 15

7-{[(Dimethylamino)methyliden]amino}-3-(β-D-ribofuranosyl)-3H-1,2,3-triazolo-[4,5-d]pyrimidin (11).

100 mg (0.37 mmol) 7-Amino-3-β-D-ribofuranosyl-(3H)-1,2,3-triazolo[4,5-d]-pyrimidin läßt man über Nacht in 2 ml trockenem DMF und 0.25 ml (1.85 mmol) N,N-Dimethylformamiddimethylacetal bei Raumtemperatur rühren. Zu dieser Reaktionsmischung gibt man anschließend 4 ml Methanol. Nach weiteren zwei Stunden Rühren wird der Ansatz zur Trockene eingedampft, mit Toluol coevaporiert und an Kieselgel chromatographiert. (Säule: 3x20 cm, Laufmittel $CH_2Cl_2$/MeOH 98:2-90:10). In der Hauptzone erhält man 0.06 g (52 %) einer farblosen, glasartigen erstarrten Substanz.
DC (Kieselgel, $CH_2Cl_2$/MeOH = 9:1): $R_f$ = 0.25
UV $\lambda_{max}$ ($\varepsilon$) = 235, 325.
$^1$H-NMR ($D_6$-DMSO) $\delta$: 3.21; 3.27 (2s, N $(CH_3)_2$); 3.55 (m, $H_2$-5'), 4.00 (m, H-4'); 4.31 (m, H-3'); 4.87 (m, H-2'); 4.96 (t, HO-5'); 5.32 (d, HO-3'); 5.60 (d, HO-2'); 6.19 (d, J = 4.7 Hz, H-1'); 8.57 (s, H-5); 9.06 (s, N = CH).

Beispiel 16

7-{[1-(Dimethylamino)ethyliden]amino}-3-(β-D-ribofuranosyl)-3H-1,2,3-triazolo-[4,5-d]pyrimidin (12).

500 mg (1.86 mmol) 9 werden in 10 ml Methanol p.a. mit 0.91 ml (5.59 mmol) N,N-Dimethylacetamid-dimethylacetal versetzt. Man läßt die Suspension 14 Stunden bei Raumtemperatur rühren. Die daraus entstandene Lösung wird am Rotationsverdampfer vom Lösungsmittel befreit und mit Toluol coevaporiert. Der Rückstand wird nochmals mit 10 ml Methanol versetzt und zwei Stunden bei Raumtemperatur gerührt. Nach dem Abtrennen des Lösungsmittels chromatographiert man den Rückstand an Kieselgel (Säule 4x20 cm, Laufmittel $CH_2Cl_2$/MeOH 98:2-90:10). Ausbeute: 0.48 g (76 %) farbloser Schaum.
DC (Kieselgel, $CH_2Cl_2$/MeOH = 9:1): $R_f$ = 0.35
UV (Methanol) $\lambda_{max}$ ($\varepsilon$) = 233 (9300), 270 (3600), 324 (26100).
$^1$H-NMR ($D_6$-DMSO) $\delta$: 2.28 (s, N=C-$CH_3$); 3.20 (s, $N(CH_3)_2$); 3.55 (m, $H_2$-5'); 4.00 (m, H-4'); 4.30 (m, H-3'); 4.86 (m, H-2'); 4.98 (t, HO-5'); 5.29 (d, J = 5.2 Hz, HO-3'); 5.57 (d, J = 5.9 Hz, HO-2'), 6.18 (d, J = 5.2 Hz, H-1'); 8.54 (s, H-5).

| | |
|---|---|
| $C_{13}H_{19}N_7O_4$ | Ber. C 46.28 H 5.69 N 29.07. |
| | Gef. C 46.45 H 5.63 N 28.97. |

Beispiel 17

7-{[1-(Dimethylamino)ethyliden]amino}-3-[5-O-(4,4'-dimethoxytriphenylmethyl)-β-D-ribofuranosyl]-3H-1,2,3-triazolo [4,5-d]pyrimidin (13).

Zur Trocknung werden 0.34 g (1.00 mmol) 12 zweimal in trockenem Pyridin abgedampft. Die Substanz wird in 4 ml trockenem Pyridin gelöst, mit 0.41 g (1.20 mmol) 4,4'-Dimethoxytritylchlorid versetzt und zwei Stunden bei 40°C gerührt. Nach dem Abkühlen auf Raumtemperatur gibt man 5 ml Methanol p.a. zu und rührt weitere 30 Minuten. Die Reaktionslösung wird auf ca. die Hälfte ihres Volumens eingeengt. Anschließend versetzt man mit 8 ml gesättigter Natriumhydrogencarbonatlösung und extrahiert viermal mit je 10 ml Methylenchlorid. Die vereinten organischen Phasen werden mit 15 ml gesättiger Natriumchloridlösung ausgeschüttelt, über Natriumsulfat getrocknet und eingedampft. Der Rückstand (0.73 g blaßgelber Schaum) wird mittels Säulenchromatographie weiter gereinigt (Säule: 2x20 cm, Kieselgel, $CH_2Cl_2$/MeOH 95:5). Ausbeute: 0.52 g (81 %) farbloser Schaum.
DC (Kieselgel, $CH_2Cl_2$/MeOH = 9:1): $R_f$ = 0.45
UV (Methanol) $\lambda_{max}$ (ε) = 234 (29900), 275 (13800), 324 (24800).
$^1$H-NMR ($D_6$-DMSO) δ: 2.24 (s, N= $CCH_3$); 3.10 (m, $H_2$-5'); 3.19 (s, $N(CH_3)_2$); 3.69 (s,$(OCH_3)_2$; 4.14 (m, H-4'); 4.51 (m, H-3'); 4.86 (m, H-2'); 5.28 (d, J= 6.2 Hz, HO-3'); 5.68 (d, J= 5.1 Hz, HO-2'); 6.25 (d, H-1'); 6.71-7.26 (m, 13 arom. H); 8.54 (s, H-5).

| | |
|---|---|
| $C_{34}H_{37}N_7O_6$ | Ber. C 63.83 H 5.84 N 15.33. |
| | Gef. C 63.64 H 5.84 N 15.31. |

Beispiel 18

7-{[1-(Dimethylamino)ethyliden]amino}-3-{5-O-(4,4'-dimethoxytriphenylmethyl)-2-O-[tris(1-methylethyl)silyl]-β-D-ribofuranosyl}-3H-1,2,3-triazolo[4,5-d]-pyrimidin (14).

0.35 g (0.55 mmol) der getrockneten Tritylverbindung 13 werden in 4 ml trockenem Pyridin vorgelegt. Zu dieser Lösung gibt man 140 mg (0.82 mmol) Silbernitrat und unter Argon 145 µl (0.69 mmol) Triisopropylchlorid, das zuvor in 5 ml Tetrahydrofuran gelöst wurde. Unter Lichtausschluß läßt man die Mischung bei Raumtemperatur rühren. Nach 24 Stunden gibt man weitere 120 µl (0.55 mmol) Triisopropylsilylchlorid zu und läßt nochmals 48 Stunden bei Raumtemperatur rühren. Das ausgefallene Silberchlorid wird abfiltriert, mit wenig Tetrahydrofuran nachgewaschen und das Filtrat mit 10 ml gesättigter Natriumhydrogencarbonatlösung versetzt. Man extrahiert viermal mit je 10 ml Dichlormethan und trocknet die vereinten organischen Phasen über Natriumsulfat. Nach dem Eindampfen zur Trockene erhält man 0.60 g schwach gelbliches Öl. Die Reinigung und Trennung der Reaktionsprodukte erfolgt über Säulenchromatographie. (Säule 3x20 cm, Kieselgel, Laufmittel Essigester/Petrolether 8:2). Aus der schneller migrierenden Hauptzone erhält man 0.31 g (71 %) farblosen Schaum.
DC (Kieselgel, Essigester/Petrolether 9:1): $R_f$ = 0.30
UV (Methanol) $\lambda_{max}$ (ε) = 234 (29600), 274 (6800), 325 (25900).
$^1$H-NMR ($D_6$-DMSO) δ: 0.83-0.97 (m, Si-$[CH(CH_3)_2)$; 2.24 (s, N = $CCH_3$), 3.10 (m, $H_2$-5'); 3.19 (s, $N(CH_3)_2$); 3.70 (s, $(O\text{-}CH_3)_2$); 4.19 (m, H-4'); 4.43 (m, H-3'); 5.21 (t, J = 4.4 Hz, H-2'); 5.27 (d, J = 6.3 Hz, HO-3'); 6.30 (d, J = 4.3 Hz, H-1'); 6.75-7.35 (m, 13 arom H); 8.53 (s, H-5).

| | |
|---|---|
| $C_{43}H_{57}N_7O_6$ | Ber. C 64.87 H 7.23 N 12.23. |
| | Gef. C 64.94 H 7.37 N 12.13. |

Beispiel 19

7-{[1-(Dimethylamino)ethyliden]amino}-3-{5-O-(4,4'-dimethoxytriphenylmethyl)-3-O-[tris(1-methylethyl)silyl]-β-D-ribofuranosyl}-3H-1,2,3-triazolo[4,5-d]-pyrimidin (15).

Aus der langsamer laufenden Zone der oben für die Verbindung 14 beschriebenen Säulenchromatographie erhält man 0.08 g (18 %) farblosen Schaum.
DC (Kieselgel, Essigester/Petrolether 9:1): $R_f$ = 0.15.
UV (Methanol) $\lambda_{max}$ (ε) = 234 (29800), 274 (7400), 327 (24700).
$^1$H-NMR ($D_6$-DMSO) δ: 0.98 (s, $Si[CH(CH_3)_2]_3$); 2.19 (s, N = $CCH_3$); 3.10 (m, $H_2$-5'); 3.18 (s, $N(CH_3)_2$); 3.68 (s, $(OCH_3)_2$); 4.17 (m, H-4'); 4.92 (m, H-3', H-2'); 5.64 (d, J = 5.1 Hz, HO-2'); 6.27 (d, J = 6.0 Hz, H-1'); 6.70-7.20 (m, 13 arom. H); 8.55 (s, H-5).

Beispiel 20

7-{[1-(Dimethylamino)ethyliden]amino}-3-{5-O-(4,4'-dimethoxytriphenylmethyl)-2-O-[tris(1-methylethyl)silyl}-β-D-ribofuranosyl}-3H-1,2,3-triazolo[4,5-d]-pyrimidin-3-O-Phosphonat, Triethylammoniumsalz (16).

Zu einer Lösung von 114 µl (1.3 mmol) Phosphortrichlorid und 1.43 ml (13.0 mmol) N-Methylmorpholin in 10 ml trockenem Dichlormethan, werden unter Argonatmosphäre, 0.67 g (9.75 mmol) 1,2,4-Triazol gegeben. Nach 30 min Rühren bei Raumtemperatur wird die Reaktionsmischung auf 0°C abgekühlt und, innerhalb von 10 min. die Silylverbindung (14), gelöst in 2.5 ml trockenem Dichlormethan, tropfenweise zugegeben. Die Reaktionsmischung wird für weitere 20 min bei 0°C gerührt und anschließend mit 1 M TBK-Puffer hydrolysiert. Die wäßrige Phase wird dreimal mit je 20 ml Dichlormethan extrahiert. Die vereinigten organischen Phasen werden über Natriumsulfat getrocknet, und zur Trockene eingedampft. Der Rückstand wird über eine Kieselgelsäule (3x10 cm, $CH_2Cl_2$/ MEOH/TEA 88:10:2) chromatographiert. Die das Produkt enthaltenden Fraktionen werden zusammen eingedampft, in 20 ml Dichlormethan aufgenommen, viermal mit jeweils 5 ml 0.1 M TBK-Puffer ausgeschüttelt, über Natriumsulfat getrocknet und vom Lösungsmittel befreit. Ausbeute 0.21 g (83 %) farbloser Schaum.
DC (Kieselgel, $CH_2Cl_2$/MeOH/TEA 88:10:2): $R_f$ = 0.6.
UV (Methanol) $\lambda_{max}$ (ε) = 234 (27300), 274 (11700), 325 (16400).
$^1$H-NMR ($D_6$-DMSO) δ: 0.75-0.95 (m, Si[CH($CH_3$)$_2$]$_3$); 1.15; 2.99 (m, ($CH_3CH_2$)$_3$N); 2.24 (s, N = $CCH_3$); 3.20 (s, N ($CH_3$)$_2$); ($H_2$-5' verdeckt); 3.69 (s, (O-$CH_3$)$_2$); 4.40 (m, H-4'); 4.79 (m, H-3'); 5.44 (m, H-2'); 5.50 u. 7.91 (d, $^1$J = 602 Hz, P-H); 6.27 (d, J = 6.0 Hz, H-1'); 6.76-7.40 (m, 13 arom. H); 8.50 (s, H-5); 10.90 (s, br, $N^+$-H).
$^{31}$P-NMR δ: 2.55 (dd, $^1P_{PH}$ = 602 $H_2$, $^3J_{PH}$ = 9.5 $H_2$).

Beispiel 21

Festphasensynthese der Oligoribonucleotide nach der Phosphonat-Methode

Die Synthese der Oligoribonucleotide wird im 1 µmol Maßstab durch Anwendung der Phosphonat-Technik an einem DNA-Synthesizer der Firma Applied Biosystems, Weiterstadt, hergestellt. Die Endoxidation erfolgt manuell.

1. Die Abspaltung der Oligoribonucleotide vom CPG-Träger wird durch sechzehnstündige Einwirkung von Ammoniak (25 % wäßrige Lösung/Ethanol 3:11) auf die Trägersäule erreicht.

2. Abspaltung der Basenschutzgruppen
Die ammoniakalische Lösung der Oligomere wird im Falle der nicht modifizierten $(AU)_6$-Sequenz im Wasserband für 16 Stunden auf 55°C erhitzt und im Falle der 8-Azaadenosin enthaltenden Dodecamere für 3 Stunden auf 40°C erwärmt. Die Lösungen werden bei Raumtemperatur zur Trockene eingedampft und mit abs. Ethanol coevaporiert.

3. Die 2'-Silylchutzgruppen wurden durch Einwirkung von einmolarer TBAF/THF-Lösung über 16 Stunden bei Raumtemperatur abgespalten.

Beispiel 22

Synthese von 5'-($z^8$A-U)$_6$-3' (17)

Das 3'-Phosphonat des 8-Azaadenosins wird zur Synthese des Oligoribonucleotids 5'-($z^8$A-U)$_6$-3' (17) eingesetzt.
Dabei wird Verbindung (16) zusammen mit 5'-(MeO)$_2$Tr,2'-t-BuMe$_2$Sigeschützten 3'-Phosphonaten des Uridins eingesetzt. Die Oligonucleotide werden an Controlled Pore Glass (CPG) von 3' in 5'-Riclitung synthetisiert,
wobei das 3'-terminal geschützte Nukleosid kovalent über einen Succinylspacer an die feste Phase gebunden ist. Zu Beginn wird die 5'Dmt-Gruppe des trägergebundenen Nucleosids mit 2.5 % Dichloressigsäure in Dichlormethan abgespalten. Anschließend erfolgt die Kupplung mit dem durch Pivaloylchlorid aktivierten Phosphonat. Zur Vermeidung von Fehlsequenzen werden nichtumgesetzte 5'-OH-Gruppen mit Isopropylphosphit umgesetzt.

Beispiel 23

Aufreinigung der Oligoribonucleotide

1.) Vorentsalzung

Mit Hilfe von Qiagen tip 500 Anionen-Austauscher Säulen. Eine ®Qiagen-Säule wird mit 5 ml 0,1 M TBK-Puffer equilibriert, mit der Oligomerlösung beladen und mit 5 ml 0,1 M TBK-Puffer gewaschen. Danach eluiert man die Oligomere mit 1 M TBK-Puffer von der Säule. Die Detektion der Produktfraktionen wird mittels UV/DC-Platten erreicht. Die oligomerhaltigen Fraktionen werden mit einer ®Speed Vac Zentrifuge unter Vakuum von den Pufferlösungen befreit.

2.) Präparative HPLC

Die Oligomeren wurden mit Hilfe der Reverse Phase-HPLC an einer RP 18 ®LiChrosorb-Säule isoliert. Dazu nimmt man das Oligomer in 400 µl einer 1 % wäßrigen Lösung von Diethylpyrocarbonat (DEPC) auf, erhitzt die Probe 2-3 min auf 95°C und kühlt schnell auf 0°C ab um die Bildung von Sekundärstrukturen zu unterbinden. DEPC ist ein Rnase-Inhibitor. Dann wird davon eine Probe (10 µl) aufgespritzt um die Retentionszeiten zu ermitteln. Anschließend gibt man die Lösung in Portionen von 50 - 100 µl auf die RP-18-Säule, trennt den Hauptpeak ab und engt die vereinigten Fraktionen auf ein Volumen von ca. 5 ml ein.

Mobile Phasen:

A: 0.1 M TEAA (steril, pH 7.5) / $CH_3CN$ 95:5
B: $CH_3CN$

System I: 20 min 0-20 % B in A
System II: 30 min 0-20 % B in A

Retentionszeiten des Oligomers 17

| Oligomer | Retentionszeit [min] | System |
|---|---|---|
| $(z^8A\text{-}U)_6$ | **28.6** | II |
| Flußrate: 1 ml/min | | |

3. Entsalzung

Die 5 ml Oligomerlösung werden auf die zuvor autoklavierte und mit 5 ml $CH_3CN$, 5 ml 0.05 M TEAA-Puffer (pH 7.0)/$CH_3CN$ 1:1, sowie mit 5 ml 0.05 M TEAA-Puffer equilibrierte Säule (Millipore, Eschborn) gegeben, mit 5 ml 0.05 M TEAA-Lösung gewaschen und mit einer Mischung von $MeOH/CH_3CN/H_2O$ 1:1:1 in Portionen von 1 ml werden die Oligoribonucleotide wieder von der Säule eluiert. Die oligomerenenthaltenden Fraktionen werden durch HPLC ermittelt. Nach Lyophilisation am ®Speed Vac Konzentrator werden die Oligoribonucleotide bei -25°C aufbewahrt.

Beispiel 24

Totalhydrolyse der Oligoribonucletide

0.2 $A_{260}$-Einleiten der Oligomere werden in 200 µl Tris-HCl-Puffer (pH 8.3) gelöst, mit 4 µg (2 µl) Schlangengift-Phosphordiesterase (Boehringer Mannheim) versetzt und 30 min bei 37°C inkubiert. Nach Zugabe von 3 µg (5 µl) alkalischer Phosphatase wird die Lösun weitere 15 min bei 37°C gehalten. Die Nucleosidzusammensetzung der Reaktionslösung wird anschließend durch HPLC bestimmt (Säule RP-18. mobile Phase: 0.1 M TEAA-Puffer/$CH_3CN$ 95: 5, Flußrate 1 ml/min)

Retentionszeiten der Nucleoside:

A = 11.4 min $z^8$A = 10.0 min I = 4.8 min U = 3.6 min

Die HPLC-Peakflächen werden durch die jeweiligen Extinktionskoeffizienten geteilt und zueinander ins Verhältnis gesetzt.
Extinktionskoeffizienten bei 260 nm:
$\varepsilon$ (A) = 15300, $\varepsilon$ ($z^8$A) = 7100, $\varepsilon$ (U) = 10200, $\varepsilon$ (I) = 7400

Beispiel 25:

Fractosil®-gebundenes $N^8$-8-Aza-2'-desoyxguanosin.

Zu einer Lösung des 3'-O-Succinats (6) aus Beispiel 6 (30 mg, 0.04 mmol) und 1,4-Dioxan/5% Pyridin (1 ml) wurden p-Nitrophenol (7 mg, 0.05 mmol) und N,N-Dicyclohexylcarbodiimid (10 mg, 0.048 mmol) gegeben. Nach 2 Stunden Rühren bei Raumtemperatur wurde das Filtrat der Lösung zu einer Suspension von Fractosil 200® (80 mg, 450 µmol/g; Merck) in DMF (1 ml) gegeben. Nach Zugabe von Triethylamin (100 µl) schüttelte man die Mischung für 4 Stunden, wobei zwischendurch noch eine Zugabe von Essigsäureanhydrid (20 µl) erfolgte. Der polymere Träger wurde abfiltriert, mit je 30 ml DMF, Ethanol und Ether gewaschen und im Vakuum getrocknet. Zur Bestimmung der Ausbeute des an polymergebundenen Nucleosids wurde die Substanz mit 0.1 M p-Toluolsulfonsäure (5 ml) in MeCN aufgenommen. Aus der Extinktion bei 498 nm konnte die Beladung des Trägers UV-spektrophotometrisch (Dmt = 70000) mit 64 µmol 8-Aza-2'-desoxyguanosin / g Fractosil® berechnet werden.

Beispiel 26:

Festphasensynthese der Oligodesoxyribonucleotide nach der Phosphonat-Methode.

Die Oligodesoxyribonucleotidsynthesen wurden im 1 µmol Maßstab mittels der Phosphonat-Technik an einem automatisierten DNA-Synthesizer Modell 380 B (Applied Biosystems, Weiterstadt) an fester Phase (CPG: ®Controlled Pore Glass), wobei das DNA-Fragment in 3'-5'-Richtung synthetisiert wurde, durchgeführt. Der Oxidationszyklus (Detritylierung, Kupplung, Cappen und Oxidation) folgte dabei einem für die Phosphonat-Chemie entwickeltem Programm [H. Köster, K. Kulikowsky, T. Liese, W. Heikens, V. Kohli, Tetrahedron 1981, 37, 363.]. Das basen- und an der 5'-Hydroxylgruppe Dmt geschützte Oligonucleotid wurde durch 25% wässerigem Ammoniak innerhalb von 30 min vom Träger abgespalten. Nach weiterer Zugabe von wässerigem Ammoniak (1 ml, 25%) wurden die Schutzgruppen an den Heterocyclen innerhalb von 24 Stunden bei 60°C abgespalten. Die Proben wurden unter Zugabe eines Tropfens Triethylamin (Vermeidung der vorzeitigen Abspaltung der 5'-OH Schutzgruppe) in einem Speed-Vac ®Konzentrator auf etwa 200 µl eingengt. Sie sind so bei -25°C einige Monate haltbar.

Beispiel 27:

Festphasensynthese der Oligodesoxyribonucleotide nach der Phosphoramidit-Methode.

Die Oligodesoxyribonucleotidsynthesen wurden im 1 µmol Maßstab mittels der Festphasen-Phosphoramidit-Technik an einem automatisierten DNA-Synthesizer Modell 380 B (Applied Biosystems, Weiterstadt) mit Hilfe von ®CPG (Controlled Pore Glass) oder ®Fractosil, das die erste Nucleosid-Einheit über das 3'-Ende gebunden hält, durchgeführt. Dabei wurden folgende Schritte durchgeführt:

1. Waschen mit Acetonitril abs.
2. Behandeln mit 3 % Trichloressigsäure in Dichlormethan
3. Waschen mit Acetonitril abs.
4. Kondensation mit 10 µmol 5'-O-Dimethoxytritynucleosid-3'-phosphorigsäure-β-cyanoethylest er-diisopropylamidit und 50 µmol Tetrazol in 0.3 ml Acetonitril abs.
5. Waschen mit Acetonitril
6. Capping mit 20 % Acetanhydrid in THF mit 40% Lutidin und 10 % Dimethylaminopyridin
7. Waschen mit Acetonitril
8. Oxidation mit Jod (1.3 gr in THF/Wasser/Pyridin; 70:20:5 =v:v:v)

Die Schritte 1 bis 8 , nachfolgend ein DNA-Reaktionszyklus genannt, wurden zum Aufbau des Oligonucleotids entsprechend der zu synthetisierenden Sequenz wiederholt, wobei in Schritt 4 jeweils das der Sequenz entsprechende

5'-O-Dimethoxytrityl(nucleobase)-3'-phosphorigsäure-β-cyanoethylester-diisopropylamidit eingesetzt wurde. Nach abgeschlossener Synthese erfolgt die Aufarbeitung wie in Beispiel 8 beschrieben.

Beispiel 28:

Synthese von d(Cz$^8$GCGCG).

Die Synhese erfolgte wie in Beispiel 25 beschrieben ausgehend von CPG-gebundenem-5'-O-Dimethoxytrityl-2'-desoyxguanosin. Die ersten drei Nucleotide werden mit kommerziell zugänglichen 5'-O-Dimethoxytrityl(nucleobase)-3'-H-phosphosphonaten durchgeführt. Zur Einführung des 8-Aza2'-desoxyguanosins wurde im vierten Kondenationszyklus das 3-[2-Desoxy-5-O-(4,4'-dimethoxytrityl)-β-D-erythro-pentofuranosyl] 5-{[(dimethylamino)methyliden]-amino}-3H-1,2,3-triazolo[4,5-d]pyrimidin-7-(6H)-on 3'(Triethylammonium Phosphonat) (4) aus Beispiel 4 eingesetzt.

Beispiel 29:

Synthese von d(Cz$^8$GCz$^8$GCG).

Die Synhese erfolgte analog wie in Beispiel 28 beschrieben, wobei zur Einführung des 8-Aza2'-desoxyguanosins im zweiten und vierten Kondenationszyklus jeweils das 3-[2-Desoxy-5-O-(4,4'-dimethoxytrityl)-β-D-erythro-pentofuranosyl] 5-{[(dimethylamino)methyliden]-amino}-3H-1,2,3-triazolo[4,5-d] pyrimidin-7-(6H)-on 3'(Triethylammonium Phosphonat) (4) aus Beispiel 4 eingesetzt wurde.

Beispiel 30:

Synthese von d(GCz$^8$GCGC).

Die Synhese erfolgte analog wie in Beispiel 29 beschrieben ausgehend von einem mit Cytidin beladenenen CPG-Träger, wobei zur Einführung des 8-Aza2'-desoxyguanosins im dritten Kondenationszyklus das 3-[2-Desoxy-5-O-(4,4'-dimethoxytrityl)-β-D-erythro-pentofuranosyl] 5-{[(dimethylamino)methyliden]-amino}-3H-1,2,3-triazolo [4,5-d ]pyrimidin-7-(6H)-on 3'(Triethylammonium Phosphonat) (4) aus Beispiel 4 eingesetzt wurde.

Beispiel 31:

Synthese von d(Tz$^8$GGGGT).

Die Synhese erfolgte analog wie in Beispiel 28 beschrieben ausgehend von CPG-gebundenem 5'-O-Dimethoxytrityl-thymidin, wobei zur Einführung des 8-Aza2'-desoxyguanosins im vierten Kondenationszyklus das 3-[2-Desoxy-5-O-(4,4'-dimethoxytrityl)-β-D-erythro-pentofurano syl] 5-{[(dimethylamino)methyliden]-amino}-3H-1,2,3-triazolo [4,5-d ]pyrimidin-7-(6H)-on 3'(Triethylammonium Phosphonat) (4) aus Beispiel 4 eingesetzt wurde.

Beispiel 32:

Synthese von d(TGz$^8$GGGT).

Die Synhese erfolgte analog wie in Beispiel 30 beschrieben ausgehend von CPG-gebundenem 5'-O-Dimethoxytrityl-thymidin, wobei zur Einführung des 8-Aza2'-desoxyguanosins im dritten Kondenationszyklus das 3-[2-Desoxy-5-O-(4,4'-dimethoxytrityl)-β-D-erythro-pentofuranosyl] 5-{[(dimethylamino)methyliden]-amino}-3H-1,2,3-triazolo [4,5-d]pyrimidin-7-(6H)-on 3'(Triethylammonium Phosphonat) (4) aus Beispiel 4 eingesetzt wurde.

Beispiel 33:

Synthese von d(Tz$^8$Gz$^8$Gz$^8$Gz$^8$GT).

Die Synthese erfolgte analog wie in Beispiel 30 beschrieben ausgehend von CPG-gebundenem 5'-O-Dimethoxytrityl-thymidin, wobei zur Einführung des 8-Aza2'-desoxyguanosins in den Kondenationszyklen eins bis vier jeweils das 3-[2-Desoxy-5-O-(4,4'-dimethoxytrityl)-β-D-erythro-pentofuranosyl] 5-{[(dimethylamino)methyliden]-amino}-3H-1,2,3-triazolo[4,5-d]pyrimidin-7-(6H)-on 3'(Triethylammonium Phosphonat) (4) aus Beispiel 4 eingesetzt wurde.

Beipiel 34:

Synthese von d(GTAz$^8$GAATTCTAG).

Die Synthese erfolgte wie in Beispiel 26 beschrieben ausgehend von CPG-gebundenem-5'-O-Dimethoxytrityl-2'-desoyxguanosin. Zur Einführung des 8-Aza2'-desoxyguanosins wurde im achten Kondenationszyklus das 3-[2-Desoxy-5-O-(4,4'-dimethoxytrityl)-β-D-erythro-pentofuranosyl] 5-{[(dimethylamino)methyliden]-amino}-3H-1,2,3-triazolo [4,5-d]pyrimidin-7-(6H)-on 3'(Triethylammonium Phosphonat) (4) aus Beispiel 4 eingesetzt.

Beispiel 35:

Aufreinigung der tritylgeschützten sowie entschützten Oligonucleotide durch HPLC.

Die Dmt-geschützten Oligomeren wurden in einem ersten Reinigungsschritt über HPLC an RP-18-Kieselgel aufgereinigt (Laufmittelsystem I) und bis zur Trockene im Vakuum bei 40°C eingedampft. Anschließende 20 minütige Behandlung mit 250 µl 80% Essigsäure führte zur Abspaltung der 5'-Tritylgruppe. In einem zweiten Reinigungsschritt wurden die nun völlig entschützten Oligomere ein zweites Mal über RP-18 HPLC gereinigt (Laufmittelsystem II). Die vereinigten Hauptzonen wurden abgedampft, der Rückstand in ca. 500 µl Wasser gelöst und über eine kurze RP-18 Säule entsalzt (Laufmittelsystem III). Nach Lyophilisation wurden die Oligomere (5-20 $A_{260}$ Einheiten) in 100 µl Wasser aufgenommen und bei -25°C gelagert.
Es wurden folgende Laufmittelsysteme bestehend aus:

- 0.1 M Triethylammoniumacetat, pH 7.0 / 5% Acetonitril (A)
- Acetonitril (B)
- Wasser (C)
- Methanol/Wasser (3:2) (D)

verwendet:

I: 20 min (0-20% B) in A
II: 20 min (15-40% B) in A
III: 15 min C, 10 min D
IV : 100% A
V : 100% B

Folgende Retentionszeiten der Oligomeren wurden beobachtet:

| Oligomer | Beispiel. | Retentionszeit [min] | Laufmittel |
|---|---|---|---|
| d(Cz$^8$GCGCG) | 10 | 15.1 (12.5) | I (II) |
| d(Cz$^8$GCz$^8$GCG) | 11 | 15.8 (12.9) | I (II) |
| d(GCz$^8$GCGC) | 12 | 15.5 (12.5) | I (II) |
| d(Tz$^8$GGGGT) | 13 | 13.4 (12.2) | I (II) |
| d(TGz$^8$GGGT) | 14 | 13.5 (12.1) | I (II) |
| d(Tz$^8$Gz$^8$Gz$^8$Gz$^8$GT) | 15 | 13.6 (12.4) | I (II) |
| d(GTAz$^8$GAATTCTAG) | 16 | 15.0 (12.4) | I (II) |

Beispiel 36:

Charakterisierung der Oligodesoxyribonucleotide durch enzymatische Hydrolyse.

0.2 $A_{260}$-Einheiten der Oligomere wurden in 0.1 M Tris/HCl-Puffer (pH 8.3, 200 µl) gelöst und mit Schlangengiftphosphodiesterase (EC 3.1.4.1, Crotallus durissus, Boehringer Mannheim; 6 µg) bei 37°C 45 min und mit alkalischer Phosphatase (EC 3.1.3.1, Kalbsleber, Boehringer Mannheim; 2 µg) 30 min bei 37°C inkubiert. Die Hydrolyseprodukte wurden mittels "Reverse Phase"-HPLC (RP-18, Laufmittel IV) bei 260 nm detektiert. Basierend auf den Peakflächen

und den Extinktionskoeffizienten der Nucleoside ($_{260}$: dA 15400, dC 7300, dG 11700, dT 8800, $z^8G_d$ 12000) konnte die Zusammensetzung der Oligodesoxyribonucleotide quantifiziert werden.

Beispiel 37:

Bestimmung der enzymatischen Hypochromizität.

Die UV-Absorption bei 260 nm von ca. 0.2 $A_{260}$-Einheiten der Oligomere wurde in 0.1 M Tris/HCl-Puffer (pH 8.3, 200 µl) vor und nach der Zugabe von Schlangengift-Phosphodiesterase (10 µg) bestimmt. Unter Berücksichtigung der Enzymabsorption ergibt sich die Hypochromizität nach der Beziehung:

$$H_{enzym.} = [(_{Monomer} - _{Oligomer})\ (_{Monomer})^{-1}] \times 100$$

Beispiel 38:

UV- und CD-spektroskopische Bestimmung der $T_m$-Werte und Berechnung der thermodynamischen Daten.

Die Ermittlung der $T_m$-Werte der Oligomere erfolgte an einem Cary 1 UV-Vis Spektrophotometer (Varian, Melbourne, Australien). Die Temperatur wurde mit 0.5°C bzw. 1.0°C pro Minute linear variiert. Zur Untersuchung der Schmelztemperatur wurden Oligomerkonzentrationen zwischen 0.2-0.8 $A_{260}$-Einheiten in 1 ml 60 mM Natrium-Cacodylat-Puffer (pH 7.5, 1 M NaCl, 100 mM $MgCl_2$) verwendet. Die Einzelstrang-Konzentration betrug bei den Experimenten an den nicht selbstkomplementären Oligonucleotiden 0.2-0.6 OD. Die Schmelzhypochromizität in % ergibt sich aus der Absorptionsänderung vor und nach dem Aufschmelzen nach folgender Gleichung:

$$H_{Schm.} = [(A_e - A_t)A_e^{-1}] \times 100$$

Die Analyse der Schmelzkurven erfolgte mit einem Programm ausgehend von einem Zweizustandsmodell ("stacked/ unstacked") entsprechend der Gleichung:

$$\ln K = \ln [(E^S - E)/(E^U - E)] = S/R - H/RT$$

mit E = Absorption bei der entsprechenden Wellenlänge, S = "stacked" und U = "unstacked"). Die temperaturabhängigen CD-Spektren wurden an einem Jasco 600 Spektropolarimeter mit temperierbarer Quarz-Küvette in einem Wellenlängenbereich von 200-350 nm aufgenommen. Die Temperaturerhöhung wurde in Intervallen von 5-10°C in einem Bereich von 5 - 80°C in Konzentrationen von 3 - 15 µM in 60 mM Na-Cacodylat-Puffer sowie bei 0.1 M, 1 und 4 M NaCl durchgeführt .

Beispiel 39

$T_m$-Werte und Hypochromizitätsdaten für die Duplexbildung a)

| Oligomer | Tm [°C] | Hypochromizität. [%] |
|---|---|---|
| d(CGCGCG) | 45 | 22 |
| d(GCGCGC) | 45 | 29 |
| d(C$z^8$GCGCG) | 49 | 23 |
| d(C$z^8$GC$z^8$GCG) | 52 | 21 |
| d(GC$z^8$GCGC) | 47 | 27 |

a) Gemessen in 1 M NaCl, 100 mM $MgCl_2$, 60 mM Cacodylatpuffer, pH 7.0.

Beispiel 40

Überprüfung auf Nuclease-Stabilität

10 nmol des zu untersuchenden Oligonucleotids werden in 450 µl 20%-igem fötalem Kälberserum in RPMI-Medium und 50 ml bidestilliertem Wasser gelöst und bei 37°C inkubiert. Dann werden sofort und nach 1, 2, 4, 7 und 24 Stunden 10 µl Proben für die Gelelektrophorese bzw. 20 µl Proben für die HPLC entnommen, zum Abbruch der Reaktion mit 5 ul bzw. 10 ul Formamid versetzt und 5 Minuten auf 95°C erhitzt. Für die Gelelektrophorese werden die Proben auf ein 15% Polyacrylamid-Gel (2% BIS) aufgetragen und dieses bei etwa 3000 Voltstunden entwickelt. Die Banden werden durch die Silberfärbung sichtbar gemacht. Zur HPLC-Analyse werden die Proben auf eine Gen-Pak Fax HPLC Säule (Waters/Millipore) gespritzt und bei 1 ml/min mit 5 bis 50 % Puffer A in B chromatographiert (Puffer A: 10mM Natrium-dihydrogenphosphat, 0,1 M NaCl in Acetonitril/Wasser 1:4 (v:v) pH 6,8; Puffer B: wie A, jedoch 1,5 M NaCl).

Beispiel 41

Testung auf antivirale Aktivität:

Die antivirale Aktivität der Prüfsubstanzen gegen verschiedene humanpathogene Herpesviren wird im Zellkulturtestsystem untersucht. Für den Versuch werden Affennierenzellen (Vero, $2\times10^5$/ml) in serumhaltigem Dulbecco's MEM (5 % Fötales Kälberserum FCS) in 96-Napf-Mikrotiterplatten ausgesät und 24 h bei 37°C und 5 % $CO_2$ inkubiert. Das serumhaltige Medium wird dann abgesaugt und die Zellen werden zweimal mit serumfreiem Dulbecco's MEM (-FCS) überspült. Die Testsubstanzen werden in $H_2O$ auf eine Konzentration von 600 µM vorverdünnt und bei -18°C aufbewahrt. Für den Test erfolgen weitere Verdünnungsschritte in Dulbecco's Minimal Essential Medium (MEM). Je 100 µl der einzelnen Prüfsubstanzverdünnungen werden zusammen mit 100 µl serumfreiem Dulbecco's MEM (-FCS) zu den gespülten Zellen gegeben. Nach 3 h Inkubation bei 37°C und 5% $CO_2$ werden die Zellen mit Herpes simplex Virus Typ 1 (ATCC VR733, HSV-1 F-strain) oder mit Herpes simplex Virus Typ 2 (ATCC VR734, HSV-2 G-Strain) infiziert in Konzentrationen, bei denen der Zellrasen innerhalb von 3 Tagen vollkommen zerstört wird. Bei HSV-1 beträgt die Infektionsstärke 500 Plaque-bildende Einheiten (PFU) pro Napf, bei HSV-2 350 PFU/Napf. Die Versuchsansätze enthalten dann Prüfsubstanz in Konzentrationen von 80 µM bis 0,04 µM in MEM, ergänzt durch 100 U/ml Penicillin G und 100 mg/l Streptomycin. Alle Versuche werden als Doppelbestimmung durchgeführt mit Ausnahme der Kontrollen, die achtfach je Platte durchgeführt werden. Die Versuchsansätze werden 17 h bei 37°C und 5 % $CO_2$ inkubiert. Die Cytotoxizität der Prüfsubstanzen wird nach 20 h Gesamtinkubationszeit durch mikroskopische Begutachtung der Zellkulturen bestimmt. Als Dosis tolerata maxima (DTM) wird die höchste Präparatkonzentration bezeichnet, die unter den genannten Versuchsbedingungen noch keine mikroskopisch erkennbaren Zellschädigungen hervorruft. Es erfolgt daraufhin die Zugabe von FCS auf eine Endkonzentration von 4 % mit weiterer Inkubation für 55 h bei 37°C und 5% $CO_2$. Die unbehandelten Infektionskontrollen zeigen dann einen kompletten cytopathischen Effekt (CPE). Nach mikroskopischer Begutachtung der Zellkulturen werden diese dann mit Neutralrot entsprechend dem Vitalfärbungsverfahren nach Finter (1966) angefärbt. Die antivirale Aktivität einer Testsubstanz wird definiert als minimale Hemmkonzentration (MHK), die benötigt wird, um 30-60 % der Zellen vor dem virusbedingten cytopathogenen Effekt zu schützen.

Abkürzungen:

| | |
|---|---|
| A | Adenosin |
| bz | Benzoyl |
| br. | breit |
| Ber. | berechnet |
| CD | Circulardichroismus |
| d | Duplett |
| DC | Dünnschichtchromatographie |
| dG | 2'-Desoxyguanosin |
| dA | 2'-Desoxyadenosin |
| dC | 2'-Desoxycytidin |
| dT | 2'-Desoxythymidin |
| DEPC | Diethylpyrocarbonat |
| DMA | Dimethylacetamidin |
| ($D_6$)DMSO | Dimethylsulfoxid, 6-fach deuteriert |

| | |
|---|---|
| DMF | Dimethylformamid |
| DNA | Desoxyribonucleinsäure |
| Dmt | 4,4'-Dimethoxytrityl, (4,4'-Dimethoxytriphenylmethyl) |
| EDTA | Ethylendiamintetraacetat |
| EtOAc | Ethylacetat |
| $Et_3N$ | Triethylamin |
| FC | Flashchromatographie |
| G | Freie Enthalpie |
| Gef. | gefunden |
| getr. | getrocknet |
| h | Stunde |
| H | Enthalpie der Duplexbildung |
| HPLC | Hochdruckflüssigkeitschromatographie |
| Hyp. | Hypochromizität |
| I | Inosin |
| ibu | Isobuturyl |
| J | Kopplungskonstante |
| $K_m$ | Michaelis-Menten-Konstante |
| NMR | Kernmagnetische Resonanz |
| PAGE | Polyacrylamidgelelektrophorese |
| PCR | Polymerase chain reaction |
| ppm | parts per million |
| 2-PrOH | Isopropanol |
| $R_f$ | Retention bei der DC relativ zur Laufmittelfront |
| RNA | Ribonucleinsäure |
| RP | Reverse-Phase |
| R.T. | Raumtemperatur |
| s | Singulett |
| S | Enthropie der Duplexbildung |
| Smp. | Schmelzpunkt |
| SVPD | Schlangengift-Phosphordiesterase |
| t | Triplett |
| TBAF | Tetrabutylammoniumfluorid |
| TBK | Triethylammoniumbicarbonat |
| TEP | Triethylammoniumphosphonat |
| Tms | Tris(1-methylethyl)silyl |
| Tris | Tris(hydroxymethyl)aminomethan |
| $T_m$ | Schmelztemperatur bei Oligomeren |
| U | Uridin |
| UV | Ultraviolett |
| $v_{max}$ | maximale Reaktionsgeschwindigkeit |
| $z^8A$ | 8-Azaadenosin |
| $z^8G$ | 8-Aza-2'-desoxyguanosin |
| $\lambda$ | Wellenlänge |
| $\in$ | molarer Extinktionskoeffizient |

## Patentansprüche

1. Oligonucleotid der Formel I

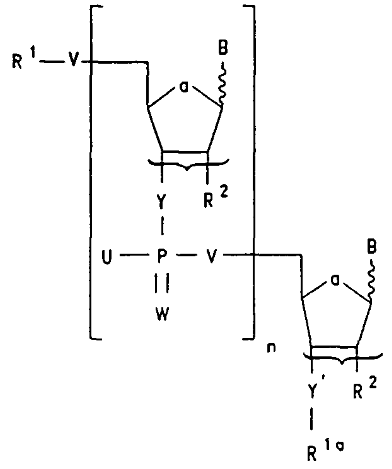

(I)

sowie dessen physiologisch verträglichen Salze worin

R1 Wasserstoff, $C_1$-$C_{18}$-Alkyl, $C_2$-$C_{18}$-Alkenyl, $C_2$-$C_{18}$-Alkinyl, $C_2$-$C_{18}$-Alkylcarbonyl, $C_3$-$C_{19}$-Alkenylcarbonyl, $C_3$-$C_{19}$-Alkinylcarbonyl, ($C_6$-$C_{14}$)-Aryl-($C_1$-$C_8$)-alkyl, eine in der Nukleotidchemie übliche Schutzgruppe oder einen Rest der Formel IIa

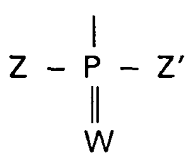

(IIa)

bedeutet;

R1a Wasserstoff, $C_1$-$C_{18}$-Alkyl, $C_2$-$C_{18}$-Alkenyl, $C_2$-$C_{18}$-Alkinyl, $C_2$-$C_{18}$-Alkylcarbonyl, $C_3$-$C_{19}$-Alkenylcarbonyl, $C_3$-$C_{19}$-Alkinylcarbonyl, ($C_6$-$C_{14}$)-Aryl-($C_1$-$C_8$)-alkyl, oder einen Rest der Formel IIb

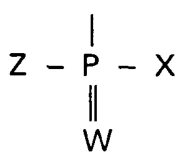

(IIb)

bedeutet;

$R^2$ Wasserstoff, Hydroxy, $C_1$-$C_{18}$-Alkoxy, $C_1$-$C_6$-Alkenyloxy, insbesondere Allyloxy, Halogen, Azido oder $NH_2$ bedeutet;

a für Oxy oder Methylen steht;

n eine ganze Zahl von 3 bis 99 bedeutet;

W Oxo, Thioxo oder Selenoxo bedeutet;

V Oxy, Sulfandiyl oder Imino bedeutet;

Y Oxy, Sulfandiyl, Imino oder Methylen bedeutet;

Y' Oxy, Sulfandiyl, Imino, $(CH_2)_m$ oder $V(CH_2)_m$ ist, worin

m eine ganze Zahl von 1 bis 18 bedeutet;

X Hydroxy oder Mercapto bedeutet;

U Hydroxy, Mercapto, SeH, $C_1$-$C_{18}$-Alkoxy, $C_1$-$C_{18}$-Alkyl, $C_6$-$C_{20}$-Aryl, ($C_6$-$C_{14}$)-Aryl-($C_1$-$C_8$)-alkyl, $NHR^3$, $NR^3R^4$ oder einen Rest der Formel III

$$(OCH_2CH_2)_pO(CH_2)_qCH_2R^5 \quad (III)$$

bedeutet, worin

$R^3$ $C_1$-$C_{18}$-Alkyl, $C_6$-$C_{20}$-Aryl, ($C_6$-$C_{14}$)-Aryl-($C_1$-$C_8$)-alkyl, 2-$(CH_2)_c$-$[NH(CH_2)_c]_d$-$NR^6R^6$, worin c eine ganze Zahl von 2 bis 6 und d eine ganze Zahl von 0 bis 6 ist, und $R^6$ unabhängig voneinander Wasserstoff oder $C_1$-$C_6$-Alkyl oder $C_1$-$C_4$-Alkoxy-$C_1$-$C_6$-alkyl ist;

$R^4$ $C_1$-$C_{18}$-Alkyl, $C_6$-$C_{20}$-Aryl oder ($C_6$-$C_{10}$)-Aryl-($C_1$-$C_8$)-alkyl bedeutet oder im Falle von $NR^3R^4$ zusammen mit $R^3$ und dem sie tragenden Stickstoffatom einen 5-6-gliedrigen heterozyklischen Ring bedeutet, der zusätzlich ein weiteres Heteroatom aus der Reihe O, S, N enthalten kann,

p eine ganze Zahl von 1 bis 100 ist,

q eine ganze Zahl von 0 bis 22 ist,

$R^5$ Wasserstoff oder eine funktionelle Gruppe wie beispielsweise Hydroxy, Amino, $C_1$-$C_{18}$-Alkylamino, COOH, $CONH_2$, COO($C_1$-$C_4$)-Alkyl oder Halogen bedeutet;

Z und Z' unabhängig voneinander

Hydroxy, Mercapto, SeH, $C_1$-$C_{22}$-Alkoxy, -O-$(CH_2)_b$-$NR^6R^7$, worin b eine ganze Zahl von 1 bis 6 ist, und $R^7$ $C_1$-$C_6$-Alkyl ist oder $R^6$ und $R^7$ zusammen mit dem sie tragenden Stickstoffatom einen 3-6 gliedrigen Ring bilden, $C_1$-$C_{18}$-Alkyl, $C_6$-$C_{20}$-Aryl, ($C_6$-$C_{14}$)-Aryl-($C_1$-$C_8$)-alkyl, ($C_6$-$C_{14}$)-Aryl-($C_1$-$C_8$)-alkoxy, wobei Aryl auch Heteroaryl bedeutet und Aryl gegebenenfalls durch 1, 2 oder 3 gleiche oder verschiedene Reste aus der Reihe Carboxy, Amino, Nitro, $C_1$-$C_4$-Alkylamino, $C_1$-$C_6$-Alkoxy, Hydroxy, Halogen und Cyano substituiert ist, $C_1$-$C_{18}$-Alkylmercapto, $NHR^3$, $NR^3R^4$, einen Rest der Formel III oder eine Gruppe bedeuten, die die intrazelluläre Aufnahme begünstigt oder als Markierung einer DNA-Sonde dient oder bei der Hybridisierung des Oligonucleotidanalogons an die Target-Nucleinsäure diese unter Bindung, Quervernetzung oder Spaltung angreift, und

die geschweifte Klammer andeutet, daß sich $R^2$ und der benachbarte Phosphoryl-Rest in 2'- und 3'-Stellung oder auch umgekehrt in 3'- und 2'-Stellung befinden können,

wobei jedes Nucleotid in seiner D- bzw. L-Konfiguration vorliegen kann und sich die Base B in α- bzw. β-Stellung befinden kann, wobei unabhängig voneinander für eine in der Nukleotidchemie übliche Base, beispielsweise für natürliche Basen wie Adenin, Cytosin, Thymin, Guanin, Uracil, Hypoxanthin oder unatürliche Basen wie beispielsweise Purin, 8-Azapurin, 2.6-Diaminopurin, 7-Deazaadenin, 7-Deazaguanin, $N^4N^4$-Ethancytosin, $N^6N^6$-Ethano-2.6-diaminopurin, Pseudoisocytosin, 5-Methylcytosin, 5-Fluoruracil, 5-($C_3$-$C_6$)-Alkinyluracil, 5-($C_3$-$C_6$)-Alkinylcytosin oder deren Prodrugformen steht, wobei mindestens ein B eine Base der Formel IV bedeutet

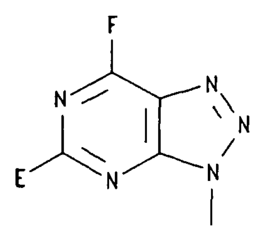

(IV)

worin E und F unabhängig voneinander H, OH oder $NH_2$ bedeuten.

2. Oligonucleotid gemäß Anspruch 1, **dadurch gekennzeichnet, daß** sich die Base B in β-Stellung befindet, die Nucleotide in der D-Konfiguration vorliegen, $R^2$ sich in 2'-Stellung befindet und a für Oxy steht.

3. Oligonucleotid gemäß Anspruch 1 oder 2, **dadurch gekennzeichnet, daß**

$R^1$ Wasserstoff, $C_1$-$C_6$-Alkyl, insbesondere Methyl, oder einen Rest der Formel IIa bedeutet;

$R^{1a}$ Wasserstoff, $C_1$-$C_6$-Alkyl, insbesondere Methyl, oder einen Rest der Formel IIb bedeutet;

$R^2$ Wasserstoff, $C_1$-$C_6$-Alkoxy, $C_1$-$C_6$-Alkenyloxy, insbesondere Allyloxy, oder Hydroxy, insbesondere Wasserstoff, bedeutet;

n eine ganze Zahl von 4 bis 39 , insbesondere 5 bis 29, bedeutet;

m eine ganze Zahl von 1 bis 6, insbesondere 1, bedeutet;

U Hydroxy, Mercapto, $C_1$-$C_6$-Alkoxy, $C_1$-$C_6$-Alkyl, $NR^3R^4$ oder $NHR^3$, insbesondere Hydroxy oder $C_1$-$C_6$-Alkyl, bedeutet, worin

$R^3$ $C_1$-$C_8$-Alkyl, bevorzugt $C_1$-$C_4$-Alkyl, oder Methoxyethyl ist, und B, W, V, Y, Y', X und Z die in Anspruch 1 aufgeführte Bedeutung haben.

**4.** Oligonucleotid gemäß einem oder mehreren der Ansprüche 1, 2 oder 3, **dadurch gekennzeichnet, daß** V, Y und Y' die Bedeutung von Oxy, Sulfandiyl oder Imino haben.

**5.** Oligonucleotid gemäß einem oder mehreren der Ansprüche 1-4, **dadurch gekennzeichnet, daß** W die Bedeutung von Oxo oder Thioxo hat.

**6.** Oligonucleotid gemäß einem oder mehreren der Ansprüche 1-5, **dadurch gekennzeichnet, daß** U die Bedeutung von Hydroxy, Methyl oder Mercapto hat.

**7.** Oligonucleotid gemäß einem oder mehreren der Ansprüche 1-6, **dadurch gekennzeichnet, daß** $R^1$ und/oder $R^{1a}$ für Wasserstoff steht.

**8.** Verfahren zur Herstellung der Oligonucleotide der Formel I sowie dessen physiologisch verträglichen Salze nach einem der Ansprüche 1-7, **dadurch gekennzeichnet, daß** man sukzessive ein Nucleotid mit jeweils einer Nucleobase an einen entsprechenden derivatisierten Träger oder an eine wachsende Oligomerkette ankondensiert.

**9.** Verwendung der Oligonucleotide nach einem der Ansprüche 1 - 7 zur Herstellung eines Arzneimittels oder Diagnostikums.

**10.** Verfahren zur Herstellung eines Arzneimittels oder Diagnostikums, **dadurch gekennzeichnet, daß** mindestens ein Oligonucleotid nach einem der Ansprüche 1 - 7 mit einem physiologisch annehmbaren Träger sowie gegebenenfalls geeigneten Zusatzstoffen und/oder Hilfsstoffen vermischt wird.

**11.** Arzneimittel oder Diagostikum, enthaltend eine oder mehrere der Verbindungen nach einem der Ansprüche 1 - 7, gegebenenfalls zusammen mit physiologisch annehmbaren Träger und/oder Hilfsstoffen.

**12.** Nucleotidmonomer der Formel V

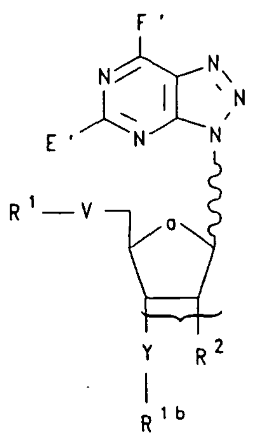

(V)

worin

V Oxy, Sulfandiyl oder Imino ist;
$Y^b$ Oxy, Sulfandiyl oder Methylen ist;
a für Oxy oder Methylen steht;
$R^{2b}$ Wasserstoff, $OR^{12}$, $C_1$-$C_{18}$-Alkoxy, $C_1$-$C_6$-Alkenyloxy, insbesondere Allyloxy, Halogen, Azido oder $NR^{10}R^{11}$ bedeutet;
$R^1$ eine in der Nukleotidchemie übliche Schutzgruppe bedeutet;
$R^{1b}$ einen Rest der Formel IIc oder IId bedeutet

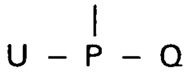 (IIc) 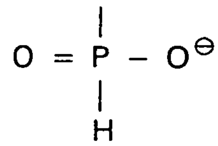 (IId)

worin

| | |
|---|---|
| U | $O-R^7$ oder $S-R^7$ bedeutet; |
| Q | einen Rest $-NR^8R^9$ bedeutet |
| $R^7$ | $-(CH_2)_2-CN$ bedeutet; |
| $R^8$ und $R^9$ | gleich oder verschieden sind und $C_1$-$C_6$-Alkyl, insbesondere Isopropyl oder Ethyl bedeuten oder zusammen mit dem sie tragenden Stickstoffatom einen 5-9-gliedrigen heterozyklischen Ring bedeuten, der zusätzlich ein weiteres Heteroatom aus der Reihe O, S, N enthalten kann, insbesondere |

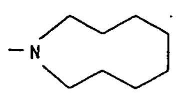 , 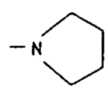 oder 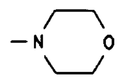 ;

| | |
|---|---|
| E' und F' | unabhängig voneinander H, OH oder $NR^{10}R^{11}$ bedeuten; |
| $R^{10}$ und $R^{11}$ | gleich oder verschieden sind und Wasserstoff oder eine in der Nukleotidchemie übliche Aminoschutzgruppe bedeuten oder $R^{10}$ und $R^{11}$ gemeinsam eine in der Nukleotidchemie übliche Aminoschutzgruppe bilden; |
| $R^{12}$ | eine in der Nukleotidchemie übliche Hydroxylschutzgruppe, wie beispielsweise t-Butyl-dimethylsilyl, Tri-isopropyl-silyl, o-nitro-Benzyl, p-nitro-Benzyl, Tris(1-methylethyl)silyl oder 2-Fluorphenyl-4-methoxypiperidin-4-yl (FPMP) bedeutet, |

und die geschweifte Klammer andeutet, daß sich $R^2$ und der benachbarte Phosphorylrest in 2'- und 3'-Stellung oder auch umgekehrt in 3'-und 2'- Stellung befinden können.

**13.** Verfahren zur Herstellung eines Nucleotidmonomers der Formel V gemäß Anspruch 12, **dadurch gekennzeichnet, daß** die korrespondierenden Nucleosidmonomere der Formel V nach Einführung geeigneter Aminoschutzgruppen bzw. Hydroxylschutzgruppen in die entsprechenden Phosphonat- oder Phosphoramiditderivate übergeführt werden.

**14.** Verwendung eines Nucleotidmonomers gemäß Anspruch 12 zur Herstellung von Oligonukleotiden, die zusammen mit ihren Target-Nucleinsäuren stabile Hybride bilden.

**15.** Verbindung der Formel VI

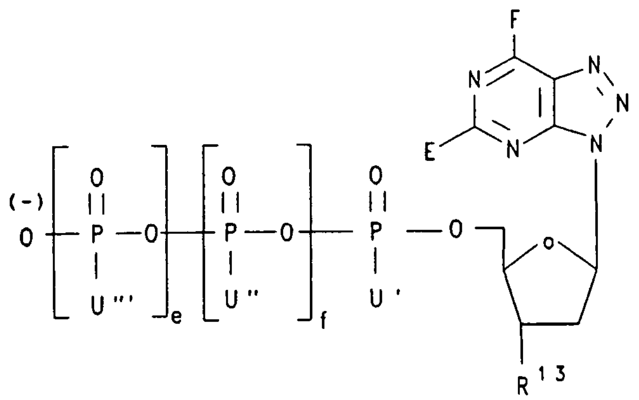

(VI)

worin unabhängig voneinander
U' = U" = U"' gleich Hydroxyl oder Mercapto ist;

| | |
|---|---|
| e und f | O oder 1 bedeuten; |
| $R^{13}$ | Wasserstoff oder OH bedeutet und |
| E und F | H, OH oder $NH_2$ bedeuten, |

wobei Verbindungen der Formel VI ausgenommen sind, in denen
U', U", U"', $R^{13}$ und F OH bedeuten, E gleich $NH_2$ ist und e und f 1 bedeuten.

**16.** Verwendung eines Nucleotidmonomers der Formel VI gemäß Anspruch 15 als Hilfsmittel in der Molekularbiologie.

**17.** Verwendung eines Oligonucleotids der Formel I gemäß einem oder mehreren der Ansprüche 1-7 als Hilfsmittel in der Molekularbiologie.